# EUROPEAN PATENT SPECIFICATION

(11) **EP 2 846 835 B1**
(45) Date of publication and mention of the grant of the patent: **06.09.2017**
(21) Application number: 13721766.7
(22) Date of filing: 10.05.2013
(51) Int. Cl.: A61K 9/20, A61K 31/135

(54) **THERMOFORMED, TAMPER-RESISTANT PHARMACEUTICAL DOSAGE FORM CONTAINING ZINC**
THERMOGEFORMTE MANIPULATIONSSICHERE, PHARMAZEUTISCHE DARREICHUNGSFORM MIT ZINK
FORME POSOLOGIQUE PHARMACEUTIQUE INVIOLABLE THERMOFORMÉE CONTENANT DU ZINC

(30) Priority: 11.05.2012 EP 12003743
(43) Date of publication of application: 18.03.2015
(73) Proprietor: Grünenthal GmbH, 52078 Aachen (DE)
(72) Inventor: BARNSCHEID, Lutz, 41239 Mönchengladbach (DE); GALIA, Eric, 52078 Aachen (DE); GEISSLER, Anja, 52222 Stolberg (DE); PÄTZ, Jana, 53332 Bornheim (DE); SCHWIER, Sebastian, 52076 Aachen (DE); BARONSKY-PROBST, Julia, 52066 Aachen (DE)
(74) Representative: Bülle, Jan
(86) International application number: PCT/EP2013/059728
(87) International publication number: WO 2013/167735

(56) References cited:
- EP-A2- 2 402 004
- WO-A1-2005/105036
- US-A1- 2006 002 860

## Description

The invention relates to a thermoformed, tamper-resistant pharmaceutical dosage form comprising a pharmacologically active ingredient; a polyalkylene oxide having a weight average molecular weight of more than 200,000 g/mol; and a zinc component, wherein the content of said zinc component is at least 1 ppm, relative to the total weight of the pharmaceutical dosage form, which has breaking strength of at least 300N, measured in accordance with the Eur. Ph. 6.0, 2.09.08.

Many pharmacologically active ingredients have a potential of being abused and thus, are advantageously provided in form of tamper resistant pharmaceutical dosage forms. Prominent examples of such pharmacologically active ingredients are opioids.

It is known that abusers crush conventional tablets, which contain opioids, to defeat the time-release "micro-encapsulation" and then ingest the resulting powder orally, intra-nasally, rectally, or by injection.

Various concepts for the avoidance of pharmacologically active ingredient abuse have been developed. One concept relies on the mechanical properties of the pharmaceutical dosage forms, particularly an increased breaking strength (resistance to crushing). The major advantage of such pharmaceutical dosage forms is that comminuting, particularly pulverization, by conventional means, such as grinding in a mortar or fracturing by means of a hammer, is impossible or at least substantially impeded.

Such pharmaceutical dosage forms are useful for avoiding pharmacologically active ingredient abuse of the pharmacologically active ingredient contained therein, as they may not be powdered by conventional means and thus, cannot be administered in powdered form, e.g. nasally. The mechanical properties, particularly the high breaking strength of these pharmaceutical dosage forms renders them tamper resistant. In the context of such tamper-resistant pharmaceutical dosage forms it can be referred to, e.g., WO 2005/016313, WO 2005/016314, WO 2005/063214, WO 2005/102286, WO 2006/002883, WO 2006/002884 (corresponding to US 2006/0002860), WO 2006/002886, WO 2006/082097, WO 2006/ 082099, WO 2008/107149, WO 2009/092601, and WO 2011/009 603.

Methods and compositions for deterring abuse of orally administered pharmaceutical products are disclosed in WO 2006/058 249 and EP 2 402 004.

US 2006/002860 discloses an abuse-proofed oral dosage form with controlled opioid release for once daily administration. The release kinetics of the pharmacologically active ingredients from such tamper-resistant dosage forms is an important factor. It is well known that depending on how a pharmaceutically pharmacologically active ingredient is formulated into a tablet its release pattern can be modified.

On the one hand, formulations providing immediate release upon oral administration have the advantage that they lead to a fast release of the pharmacologically active ingredient in the gastrointestinal tract. As a result, a comparatively high dose of the pharmacologically active ingredient is quickly absorbed leading to high plasma levels within a short period of time and resulting in a rapid onset of medicinal action, i.e. medicinal action begins shortly after administration. At the same time, however, a rapid reduction in the medicinal action is observed, because metabolization and/or excretion of the pharmacologically active ingredient cause a decrease of plasma levels. For that reason, formulations providing immediate release of pharmacologically active ingredients typically need to be administered frequently, e.g. six times per day. This may cause comparatively high peak plasma pharmacologically active ingredient concentrations and high fluctuations between peak and trough plasma pharmacologically active ingredient concentrations which in turn may deteriorate tolerability.

Controlled release (e.g. delayed release, prolonged release, sustained release, and the like) may be based upon various concepts such as coating the pharmaceutical dosage form with a controlled release membrane, embedding the pharmacologically active ingredient in a matrix, binding the pharmacologically active ingredient to an ion-exchange resin, forming a complex of the pharmacologically active ingredient, and the like. In this context it can be referred to, e.g., W.A. Ritschel, Die Tablette, 2. Auflage, Editio Cantor Verlag Aulendorf, 2002.

In comparison to formulations providing immediate release, formulations providing prolonged release upon oral administration have the advantage that they need to be administered less frequently, typically once daily or twice daily. This can reduce peak plasma pharmacologically active ingredient concentrations and fluctuations between peak and trough plasma pharmacologically active ingredient concentrations which in turn may improve tolerability.

The ideal goal in designing a prolonged-release system is to deliver the pharmacologically active ingredient to the desired site at a rate according to the needs of the body. In the absence of feed-back control, one is left with a simple prolonging effect, where the pivotal question is at what rate a pharmacologically active ingredient should be delivered to maintain a constant blood pharmacologically active ingredient level. This constant rate should be the same as that achieved by continuous intravenous infusion where a pharmacologically active ingredient is provided to the patient at a constant rate just equal to its rate of elimination. This implies that the rate of delivery must be independent from the amount of pharmacologically active ingredient remaining in the pharmaceutical dosage form and constant over time.

A perfectly invariant pharmacologically active ingredient blood or tissue level versus time profile is the ideal starting goal of a prolonged-release system. The way to achieve this, in the simplest case, is use of a maintenance dose that releases its pharmacologically active ingredient by zero-order kinetics.

US 5,082,668 discloses an osmotically driven dosage form, namely a device comprising a wall that surrounds a compartment. The compartment comprises a beneficial agent composition and a push composition. A passageway in the wall connects the compartment with the exterior of the device for delivering the beneficial agent at a rate governed, in combination, by the wall, the beneficial agent composition and the push composition through the passageway of the device over time.

US 7,300,668 relates to a dosage form comprising: a three-dimensionally printed innermost region comprising a first regional concentration of at least one active pharmaceutical ingredient; and plural three-dimensionally printed non-innermost regions in nested arrangement and comprising: a) one or more nested internal regions, wherein an internal region completely surrounds and is in contact with the innermost regions, and any other internal region present completely surrounds another internal region located to the interior thereof; and b) an outermost region completely surrounding an internal region, wherein the internal and outermost regions are in nested arrangement, wherein the at least one active pharmaceutical ingredient is released in approximately a zero-order release.

WO 2008/086804 discloses abuse resistant polyglycol-based pharmaceutical compositions. The composition contains one or more polyglycols and one or more active substances and it is resistant to crushing, melting and/or extraction. Moreover, such compositions have the same or lower solubility in ethanolic-aqueous medium, i.e. they are not subject to ethanol-induced dose dumping effect.

WO 2008/148798 discloses a layered pharmaceutical composition suitable for oral use in the treatment of diseases where absorption takes place over a large part of the gastrointestinal tract.

WO 03/024426 discloses a controlled release pharmaceutical composition for oral use comprising a solid dispersion of: i) at least one therapeutically, prophylactically and/or diagnostically active substance, which at least partially is in an amorphous form, ii) a pharmaceutically acceptable polymer that has plasticizing properties, and iii) optionally, a stabilizing agent, the at least one active substance having a limited water solubility, and the composition being designed to release the active substance with a substantially zero order release. Zero order release is provided by a coating that remains intact during the release phase and covers the matrix composition in such a manner that only a specific surface area is subject to erosion. Thereby the surface area from which the active substance is released is kept substantially constant during the time period.

WO 2010/057036 discloses a solid composition and methods for making and using the solid composition are provided. The solid composition comprises: (a) at least one active agent with a solubility of less than about 0.3 mg/ml in an aqueous solution with a pH of at most about 6.8 at a temperature of about 37°C; and (b) a hydrophilic polymer matrix composition comprising: i) a hydrophilic polymer selected from the group consisting of METHOCEL^{®}, POLYOX^{®} WSR 1105 and combinations thereof; and optionally ii) a hydrophobic polymer selected from the group consisting of Ethocel 20 premium; and (c) an alkalizer selected from the group consisting of calcium carbonate, magnesium oxide heavy and sodium bicarbonate; wherein the composition provides at least about 70% release of the active between about 7 to about 12 hours following oral administration.

WO 2005/105036 discloses controlled release oral pharmaceutical mucoadhesive matrix formulation containing a therapeutically effective amount of tolterodine or its pharmaceutically acceptable salts, prodrugs and metabolites thereof dispersed in a rate controlling polymeric matrix comprising (1) a pH independent gelling polymer, such as polyethylene oxide, (2) a pH dependent gelling polymer, such as sodium of carboxymethylcellulose, (3) a film coating polymer component, such as Eudragit RS100 and other conventional tablet functional excipients.

V. Pillay et al., Journal of Controlled Release, 67 (2000) 67-78 disclose an approach for constant rate delivery of highly soluble bioactives from a simple monolithic system prepared by direct compression at ambient conditions.

M.E. McNeill et al., J Biomater Sci Polym 1996, 7(11), 953-63 relate to properties controlling the diffusion and release of water-soluble solutes from poly(ethylene oxide) hydrogels. Part 4 deals with extended constant rate release from partly-coated spheres.

D. Henrist *et al.* relate to *in vitro* and *in vivo* evaluation of starch-based hot stage extruded double matrix systems. The objective of developing a double matrix system consisting of a hot stage extruded starch pipe surrounding a hot stage extruded and drug-containing starch core, was to obtain a monolithic matrix system applicable in the domain of sustained drug release. The behaviour of the systems was evaluated through dissolution testing and through a randomised crossover bioavailability study on nine male volunteers. All double matrix systems showed *in vitro* a nearly constant drug release profile after an initial slower release phase of 4 h. This initial slower release phase was avoided by loading the starch pipe with a small amount of drug.

L. Yang et al., J. Pharm. Sciences, 85(2), 1996, 170-173 relate to zero-order release kinetics from a self-correcting floatable asymmetric configuration drug delivery system.

It is an object of the invention to provide pharmaceutical dosage forms having advantages compared to pharmaceutical dosage forms of the prior art.

This object has been achieved by the subject-matter of the patent claims.

It has been surprisingly found that polyalkylene oxide compositions which are composed of a polyalkylene oxide having a weight average molecular weight of more than 200,000 g/mol and a zinc component are useful in the manufacture of pharmaceutical dosage forms and have advantages compared to the polyalkylene oxide compositions of the prior art. In particular, it has been surprisingly found that aqueous dispersions of polyalkylene oxide compositions which are composed of a polyalkylene oxide having a weight average molecular weight of more than 200,000 g/mol and a zinc component have lower pH values than aqueous dispersions of polyakylene oxide compositions containing substantially no zinc component. It appears that the lower pH value can enhance the stability of the pharmacologically active ingredients and the polyalkylene oxide polymers that are contained in the pharmaceutical dosage forms. Thus, there is evidence that polyalkylene oxide compositions which are composed of a polyalkylene oxide having a weight average molecular weight of more than 200,000 g/mol and a zinc component have positive effects on the shelf life of the pharmaceutical dosage forms.

A first aspect of the invention relates to a thermoformed, tamper-resistant pharmaceutical dosage form comprising
a) a pharmacologically active ingredient;
b) a polyalkylene oxide having a weight average molecular weight of more than 200,000 g/mol; and
c) a zinc component, wherein the content of said zinc component is at least 1 ppm by weight, relative to the total weight of the pharmaceutical dosage form;
which has breaking strength of at least 300N, measured in accordance with the Eur. Ph. 6.0, 2.09.08.
The pharmaceutical dosage form according to the invention contains a pharmacologically active ingredient.

For the purpose of specification, the term "pharmacologically active ingredient" may refer to either one or more pharmacologically active ingredients. There are generally no limitations as to the pharmacologically active ingredient (pharmacologically active compound) which can be incorporated into the pharmaceutical dosage form according to the invention.

In one aspect the pharmaceutical dosage form contains only a single pharmacologically active ingredient. In another aspect the pharmaceutical dosage form contains a combination of two or more pharmacologically active ingredients.

Preferably, the pharmacologically active ingredient has potential for being abused. Pharmacologically active ingredients with potential for being abused are known to the person skilled in the art and comprise e.g. tranquillizers, stimulants, barbiturates, narcotics, opioids or opioid derivatives.

Preferably, the pharmacologically active ingredient exhibits psychotropic action.

Preferably, the pharmacologically active ingredient is selected from the group consisting of opioids, stimulants, tranquilizers, and other narcotics.

Particularly preferably, the pharmacologically active ingredient is an opioid. According to the ATC index, opioids are divided into natural opium alkaloids, phenylpiperidine derivatives, diphenylpropylamine derivatives, benzomorphan derivatives, oripavine derivatives, morphinan derivatives and others.

The following opioids, tranquillizers or other narcotics are substances with a psychotropic action, i.e. have a potential of abuse, and hence are preferably contained in the pharmaceutical dosage form according to the invention: alfentanil, allobarbital, allylprodine, alphaprodine, alprazolam, amfepramone, amphetamine, amphetaminil, amobarbital, anileridine, apocodeine, axomadol, barbital, bemidone, benzylmorphine, bezitramide, bromazepam, brotizolam, buprenorphine, butobarbital, butorphanol, camazepam, carfentanil, cathine/D-norpseudoephedrine, chlordiazepoxide, clobazam clofedanol, clonazepam, clonitazene, clorazepate, clotiazepam, cloxazolam, cocaine, codeine, cyclobarbital, cyclorphan, cyprenorphine, delorazepam, desomorphine, dextromoramide, dextropropoxyphene, dezocine, diampromide, diamorphone, diazepam, dihydrocodeine, dihydromorphine, dihydromorphone, dimenoxadol, dimephetamol, dimethylthiambutene, dioxaphetylbutyrate, dipipanone, dronabinol, eptazocine, estazolam, ethoheptazine, ethylmethylthiambutene, ethyl loflazepate, ethylmorphine, etonitazene, etorphine, faxeladol, fencamfamine, fenethylline, fenpipramide, fenproporex, fentanyl, fludiazepam, flunitrazepam, flurazepam, halazepam, haloxazolam, heroin, hydrocodone, hydromorphone, hydroxypethidine, isomethadone, hydroxymethylmorphinan, ketazolam, ketobemidone, levacetylmethadol (LAAM), levomethadone, levorphanol, levophenacylmorphane, levoxemacin, lisdexamfetamine dimesylate, lofentanil, loprazolam, lorazepam, lormetazepam, mazindol, medazepam, mefenorex, meperidine, meprobamate, metapon, meptazinol, metazocine, methylmorphine, metamphetamine, methadone, methaqualone, 3-methylfentanyl, 4-methylfentanyl, methylphenidate, methylphenobarbital, methyprylon, metopon, midazolam, modafinil, morphine, myrophine, nabilone, nalbuphene, nalorphine, narceine, nicomorphine, nimetazepam, nitrazepam, nordazepam, norlevorphanol, normethadone, normorphine, norpipanone, opium, oxazepam, oxazolam, oxycodone, oxymorphone, Papaver somniferum, papaveretum, pernoline, pentazocine, pentobarbital, pethidine, phenadoxone, phenomorphane, phenazocine, phenoperidine, piminodine, pholcodeine, phenmetrazine, phenobarbital, phentermine, pinazepam, pipradrol, piritramide, prazepam, profadol, proheptazine, promedol, properidine, propoxyphene, remifentanil, secbutabarbital, secobarbital, sufentanil, tapentadol, temazepam, tetrazepam, tilidine (cis and trans), tramadol, triazolam, vinylbital, N-(1-methyl-2-piperidinoethyl)-N-(2-pyridyl)propionamide, (1R,2R)-3-(3-dimethylamino-1-ethyl-2-methyl-propyl)phenol, (1R,2R,4S)-2-(dimethylamino)-methyl-4-(p-fluorobenzyloxy)-1-(m-methoxyphenyl)cyclohexanol, (1R,2R)-3-(2-dimethylaminomethyl-cyclohexyl)phenol, (1S,2S)-3-(3-dimethylamino-1-ethyl-2-methyl-propyl)phenol, (2R,3R)-1-dimethylamino-3(3-methoxyphenyl)-2-methyl-pentan-3-ol, (1 RS,3RS,6RS)-6-dimethylaminomethyl-1-(3-methoxyphenyl)-cyclohexane-1,3-diol, preferably as racemate, 3-(2-dimethylaminomethyl-1-hydroxy-cyclohexyl)phenyl 2-(4-isobutyl-phenyl)propionate, 3-(2-dimethylaminomethyl-1-hydroxy-cyclohexyl)phenyl 2-(6-methoxy-naphthalen-2-yl)propionate, 3-(2-dimethylaminomethyl-cyclohex-1-enyl)-phenyl 2-(4-isobutyl-phenyl)propionate, 3-(2-dimethylaminomethyl-cyclohex-1-enyl)-phenyl 2-(6-methoxy-naphthalen-2-yl)propionate, (RR-SS)-2-acetoxy-4-trifluoromethyl-benzoic acid 3-(2-dimethylaminomethyl-1-hydroxycyclohexyl)-phenyl ester, (RR-SS)-2-hydroxy-4-trifluoromethyl-benzoic acid 3-(2-dimethylaminomethyl-1-hydroxy-cyclohexyl)-phenyl ester, (RR-SS)-4-chloro-2-hydroxybenzoic acid 3-(2-dimethylaminomethyl-1-hydroxy-cyclohexyl)-phenyl ester, (RR-SS)-2-hydroxy-4-methyl-benzoic acid 3-(2-dimethylaminomethyl-1-hydroxy-cyclohexyl)-phenyl ester, (RR-SS)-2-hydroxy-4-methoxy-benzoic acid 3-(2-dimethylaminomethyl-1-hydroxycyclohexyl)-phenyl ester, (RR-SS)-2-hydroxy-5-nitro-benzoic acid 3-(2-dimethylaminomethyl-1-hydroxy-cyclohexyl)-phenyl ester, (RR-SS)-2',4'-difluoro-3-hydroxy-biphenyl-4-carboxylic acid 3-(2-dimethylaminomethyl-1-hydroxy-cyclohexyl)-phenyl ester, and corresponding stereoisomeric compounds, in each case the corresponding derivatives thereof, physiologically acceptable enantiomers, stereoisomers, diastereomers and racemates and the physiologically acceptable derivatives thereof, e.g. ethers, esters or amides, and in each case the physiologically acceptable compounds thereof, in particular the acid or base addition salts thereof and solvates, e.g. hydrochlorides.

In one aspect, the pharmaceutical dosage form according to the invention contains an opioid selected from the group consisting of DPI-125, M6G (CE-04-410), ADL-5859, CR-665, NRP290 and sebacoyl dinalbuphine ester. Preferrably, the pharmaceutical dosage form according to the invention contains one pharmacologically active ingredient or more pharmacologically active ingredients selected from the group consisting of oxymorphone, hydromorphone, morphine and the physiologically acceptable salts thereof.

In another aspect, the pharmacologically active ingredient is selected from the group consisting of tapentadol, faxeladol, axomadol and the physiologically acceptable salts thereof.

In still another aspect, the pharmacologically active ingredient is selected from the group consisting of 1,1-(3-dimethylamino-3-phenylpentamethylene)-6-fluoro-1,3,4,9-tetrahydropyrano[3,4-b]indole, particularly its hemicitrate; 1,1-[3-dimethylamino-3-(2-thienyl)-pentamethylene]-1,3,4,9-tetrahydropyrano[3,4-b]indole, particularly its citrate; and 1,1-[3-dimethylamino-3-(2-thienyl)pentamethylene]-1,3,4,9-tetrahydropyrano[3,4-b]-6-fluoroindole, particularly its hemicitrate. These compounds are known from, e.g., WO 2004/043967, WO 2005/066183.

The pharmacologically active ingredient may be present in form of a physiologically acceptable salt, e.g. physiologically acceptable acid addition salt.

Physiologically acceptable acid addition salts comprise any acid addition salts which can conveniently be obtained by treating the base form of the pharmacologically active ingredient with appropriate organic and inorganic acids. Pharmacologically active ingredients containing an acidic proton may be converted into their non-toxic metal or amine addition salt forms by treatment with appropriate organic and inorganic bases. The term addition salt also comprises the hydrates and solvent addition forms which the pharmacologically active ingredients are able to form. Examples of such forms are e.g. hydrates, alcoholates and the like.

Unless explicitly stated otherwise, all amounts of the pharmacologically active ingredient specified in the following are given according to the corresponding amount of the free compound.

The pharmacologically active ingredient is present in the pharmaceutical dosage form in a therapeutically effective amount. The amount that constitutes a therapeutically effective amount varies according to the pharmacologically active ingredients being used, the condition being treated, the severity of said condition, the patient being treated, and whether the pharmaceutical dosage form is designed for an immediate or retarded release. The amount of pharmacologically active ingredient(s) used in the present invention preferably ranges from about 0.01 wt.-% to about 95 wt.-%, more preferably from about 0.1 wt.-% to about 80 wt.-%, even more preferably from about 1.0 wt.-% to about 50 wt.-%, yet more preferably from about 1.5 wt.-% to about 30 wt.-%, and most preferably from about 2.0 wt.-% to 20 wt.-%, based on the total weight of the pharmaceutical dosage form.

The content of the pharmacologically active ingredient in the pharmaceutical dosage form is not limited. The dose of the pharmacologically active ingredient which is adapted for administration preferably is in the range of 0.1 mg to 2,000 mg or 0.1 mg to 1,000 mg or 0.1 mg to 500 mg, more preferably in the range of 1.0 mg to 400 mg, even more preferably in the range of 5.0 mg to 300 mg, and most preferably in the range of 10 mg to 250 mg. In a preferred embodiment, the total amount of the pharmacologically active ingredient which is contained in the pharmaceutical dosage form is within the range of from 0.01 to 200 mg, more preferably 0.1 to 190 mg, still more preferably 1.0 to 180 mg, yet more preferably 1.5 to 160 mg, most preferably 2.0 to 100 mg and in particular 2.5 to 80 mg. In another preferred embodiment, the total amount of the pharmacologically active ingredient that is contained in the pharmaceutical dosage form is within the range of from 10 to 500 mg, more preferably 12 to 450 mg, still more preferably 14 to 400 mg, yet more preferably 16 to 350 mg, most preferably 18 to 325 mg and in particular 20 to 300 mg.

Preferably, the content of the pharmacologically active ingredient is within the range of from 0.01 to 80 wt.-%, more preferably 0.1 to 50 wt.-%, still more preferably 1 to 25 wt.-%, based on the total weight of the pharmaceutical dosage form. In a preferred embodiment, the content of pharmacologically active ingredient is within the range of from 7±6 wt.-%, more preferably 7±5 wt.-%, still more preferably 5±4 wt.-%, 7±4 wt.-% or 9±4 wt.-%, most preferably 5±3 wt.-%, 7±3 wt.-% or 9±3 wt.-%, and in particular 5±2 wt.-%, 7±2 wt.-% or 9±2 wt.-%, based on the total weight of the pharmaceutical dosage form. In another preferred embodiment, the content of pharmacologically active ingredient is within the range of from 11±10 wt.-%, more preferably 11±9 wt.-%, still more preferably 9±6 wt.-%, 11±6 wt.-%, 13±6 wt.-% or 15±6 wt.-%, most preferably 11±4 wt.-%, 13±4 wt.-% or 15±4 wt.-%, and in particular 11±2 wt.-%, 13±2 wt.-% or 15±2 wt.-%, based on the total weight of the pharmaceutical dosage form. In a further preferred embodiment, the content of pharmacologically active ingredient is within the range of from 20±6 wt.-%, more preferably 20±5 wt.-%, still more preferably 20±4 wt.-%, most preferably 20±3 wt.-%, and in particular 20±2 wt.-%, based on the total weight of the pharmaceutical dosage form.

In one aspect, the pharmacologically active ingredient is contained in the pharmaceutical dosage form in an amount of 7.5±5 mg, 10±5 mg, 20±5 mg, 30±5 mg, 40±5 mg, 50±5 mg, 60±5 mg, 70±5 mg, 80±5 mg, 90±5 mg, 100±5 mg, 110±5 mg, 120±5 mg, 130±5, 140±5 mg, 150±5 mg, 160±5 mg, 170±5 mg, 180±5 mg, 190±5 mg, 200±5 mg, 210±5 mg, 220±5 mg, 230±5 mg, 240±5 mg, or 250±5 mg. In another preferred embodiment, the pharmacologically active ingredient is contained in the pharmaceutical dosage form in an amount of 5±2.5 mg, 7.5±2.5 mg, 10±2.5 mg, 15±2.5 mg, 20±2.5 mg, 25±2.5 mg, 30±2.5 mg, 35±2.5 mg, 40±2.5 mg, 45±2.5 mg, 50±2.5 mg, 55±2.5 mg, 60±2.5 mg, 65±2.5 mg, 70±2.5 mg, 75±2.5 mg, 80±2.5 mg, 85±2.5 mg, 90±2.5 mg, 95±2.5 mg, 100±2.5 mg, 105±2.5 mg, 110±2.5 mg, 115±2.5 mg, 120±2.5 mg, 125±2.5 mg, 130±2.5 mg, 135±2.5 mg, 140±2.5 mg, 145±2.5 mg, 150±2.5 mg, 155±2.5 mg, 160±2.5 mg, 165±2.5 mg, 170±2.5 mg, 175±2.5 mg, 180±2.5 mg, 185±2.5 mg, 190±2.5 mg, 195±2.5 mg, 200±2.5 mg, 205±2.5 mg, 210±2.5 mg, 215±2.5 mg, 220±2.5 mg, 225±2.5 mg, 230±2.5 mg, 235±2.5 mg, 240±2.5 mg, 245±2.5 mg, or 250±2.5 mg. In still another preferred embodiment, the pharmacologically active ingredient is contained in the pharmaceutical dosage form in an amount of 250±10 mg, 275±10 mg, 300±10 mg, 325±10 mg, 350±10 mg, 375±10 mg, 400±10 mg, 425±10 mg, 450±10 mg, 475±10 mg, 500±10 mg, 525±10 mg, 550±10 mg, 575±10 mg or 600±10 mg.

Preferably, the pharmaceutically dosage form provides a release of the pharmacologically active ingredient after 1 hour of preferably at most 60 %, more preferably at most 40 %, yet more preferably at most 30 %, still more preferably at most 20 % and most preferably at most 17%; after 2 hours preferably at most 80 %, more preferably at most 60 %, yet more preferably at most 50 %, still more preferably at most 40 % and most preferably at most 32%; after 3 hours preferably at most 85 %, more preferably at most 65 %, yet more preferably at most 55 %, still more preferably at most 48 % and most preferably at most 42%; after 4 hours preferably at most 90 %, more preferably at most 75 %, yet more preferably at most 65 %, still more preferably at most 55 % and most preferably at most 49%; after 7 hours preferably at most 95 %, more preferably at most 85 %, yet more preferably at most 80 %, still more preferably at most 70 % and most preferably at most 68%; after 10 hours preferably at most 99 %, more preferably at most 90 %, yet more preferably at most 88 %, still more preferably at most 83 % and most preferably at most 80%; and after 13 hours preferably at most 99 %, more preferably at most 95 %, yet more preferably at most 93 %, still more preferably at most 91 % and most preferably at most 89%.

In a particularly preferred aspect, the pharmacologically active ingredient is tapentadol, preferably its HCl salt, and the pharmaceutical dosage form is adapted for administration once daily or twice daily. In this aspect, the pharmacologically active ingredient is preferably contained in the pharmaceutical dosage form in an amount of from 25 to 250 mg.

In another particularly preferred aspect, the pharmacologically active ingredient is oxymorphone, preferably its HCl salt, and the pharmaceutical dosage form is adapted for administration twice daily. The pharmacologically active ingredient is preferably contained in the pharmaceutical dosage form in an amount of from 5 to 40 mg. The pharmacologically active ingredient is preferably oxymorphone, preferably its HCl salt, and the pharmaceutical dosage form is adapted for administration once daily. The pharmacologically active ingredient is preferably contained in the pharmaceutical dosage form in an amount of from 10 to 80 mg.

In another particularly preferred aspect, the pharmacologically active ingredient is oxycodone, preferably its HCl salt, and the pharmaceutical dosage form is adapted for administration twice daily. The pharmacologically active ingredient is preferably contained in the pharmaceutical dosage form in an amount of from 5 to 80 mg. The pharmacologically active ingredient may be oxycodone, preferably its HCl salt, and the pharmaceutical dosage form is adapted for administration once daily. The pharmacologically active ingredient is preferably contained in the pharmaceutical dosage form in an amount of from 10 to 320 mg. The pharmacologically active ingredient may be hydromorphone, preferably its HCl salt, and the pharmaceutical dosage form is adapted for administration twice daily. The pharmacologically active ingredient is preferably contained in the pharmaceutical dosage form in an amount of from 2 to 52 mg. The pharmacologically active ingredient may be hydromorphone, preferably its HCl salt, and the pharmaceutical dosage form is adapted for administration once daily. The pharmacologically active ingredient is preferably contained in the pharmaceutical dosage form in an amount of from 4 to 104 mg.

In yet another particularly preferred aspect, the pharmacologically active ingredient is tramadol, preferably its HCl salt, and the pharmaceutical dosage form is adapted for administration twice daily. In this aspect, the pharmacologically active ingredient is preferably contained in the pharmaceutical dosage form in an amount of from 5 to 300 mg. In another particularly preferred aspect, the pharmacologically active ingredient is tramadol, preferably its HCl salt, and the pharmaceutical dosage form is adapted for administration once daily. The pharmacologically active ingredient is preferably contained in the pharmaceutical dosage form in an amount of from 10 to 500 mg.

The pharmaceutical dosage form according to the invention is characterized by excellent durability of the pharmacologically active ingredient. Preferably, after storage for 4 weeks, more preferably 6 months, at 40°C and 75% rel. humidity, the content of pharmacologically active ingredient amounts to at least 98.0%, more preferably at least 98.5%, still more preferably at least 99.0%, yet more preferably at least 99.2%, most preferably at least 99.4% and in particular at least 99.6%, of its original content before storage.

Furthermore, the pharmaceutical dosage form according to the invention is characterized by excellent durability of the polyalkylene oxide. Preferably, after storage for 6 months at 40°C and 75% rel. humidity, the content of polyalkylene oxide amounts to at least 98.0%, more preferably at least 98.5%, still more preferably at least 99.0%, yet more preferably at least 99.2%, most preferably at least 99.4% and in particular at least 99.6%, of its original content before storage.

Suitable parameters for observing the degradation of polyalkylene oxide upon storage include the viscosity of an aqueous gel of the pharmaceutical dosage form or the content of antioxidant in the pharmaceutical dosage form, if applicable. When the polymer chains of the polyalkylene oxide deteriorate, the viscosity of an aqueous gel of the pharmaceutical dosage form decreases. The aqueous gel of the pharmaceutical dosage form is preferably prepared as described in the experimental section. Since the degradation of polyalkylene oxide is an oxidative process, it may also be followed by a decrease of the content of antioxidant, if applicable.

In a preferred embodiment, after storage for 6 months at 40°C and 75% rel. humidity, the viscosity of the aqueous gel of the pharmaceutical dosage form according to the invention decreases by at most 15%, more preferably at most 12%, still more preferably at most 10%, even more preferably at most 8%, yet more preferably at most 7 or 6%, most preferably at most 4% and in particular at most 2 or 1% with respect to the viscosity of the aqueous gel of the pharmaceutical dosage form before storage. Generally, when the pharmaceutical dosage form according to the invention contains an antioxidant, preferably α-tocopherol, the relative weight content of antioxidant in the pharmaceutical dosage form after storage for 6 months at 40°C and 75% rel. humidity, decreases by at most 19 wt.-%, more preferably at most 18 wt.-%, still more preferably at most 17 wt.-%, even more preferably at most 16 wt.-%, yet more preferably at most 15 wt.-%, most preferably at most 10 wt.-% and in particular at most 5 wt.-% with respect to the relative weight content of antioxidant in the pharmaceutical dosage form before storage.

Suitable methods for measuring the content of the pharmacologically active ingredient, the polyalkylene oxide and antioxidant in the pharmaceutical dosage form are known to the skilled artisan. In this regard it is referred to the Eur. Ph. or the USP, especially to reversed phase HPLC analysis. Preferably, the pharmaceutical dosage form is stored in closed, preferably sealed containers, preferably as described in the experimental section, most preferably being equipped with an oxygen scavenger, in particular with an oxygen scavenger that is effective even at low relative humidity. Generally, the pharmaceutical dosage form according to the invention displays improved durability of the pharmacologically active ingredient and/or the polyalkylene oxide in particular at accelerated storage conditions at 40°C and 75% r.h., while the improved durability of the pharmacologically active ingredient and/or the polyalkylene oxide is preferably less pronounced at milder storage conditions, such as at 25°C/60% r.h. or at 30°C/65% r.h.

The pharmaceutical dosage form according to the invention contains a polyalkylene oxide and a zinc component.

Preferably, the polyalkylene oxide is selected from polymethylene oxide, polyethylene oxide and polypropylene oxide, or copolymers or mixtures thereof.

The polyalkylene oxide has a weight average molecular weight (M_{W}), preferably also a viscosity average molecular weight (M_{η}) of more than 200,000 g/mol or at least 500,000 g/mol, preferably at least 1,000,000 g/mol or at least 2,500,000 g/mol, more preferably in the range of about 1,000,000 g/mol to about 15,000,000 g/mol, and most preferably in the range of about 5,000,000 g/mol to about 10,000,000 g/mol. Suitable methods to determine M_{W} and M_{η} are known to a person skilled in the art. M_{η} is preferably determined by rheological measurements, whereas M_{W} can be determined by gel permeation chromatography (GPC).

Preferably, the molecular weight dispersity M_{w}/Mₙ of the polyalkylene oxide is within the range of 2.5±2.0, more preferably 2.5±1.5, still more preferably 2.5±1.0, yet more preferably 2.5±0.8, most preferably 2.5±0.6, and in particular 2.5±0.4.

The polyalkylene oxide preferably has a viscosity at 25°C of 30 to 17,600 mPa·s, more preferably 55 to 17,600 mPa·s, still more preferably 600 to 17,600 mPa·s, yet more preferably 4,500 to 17,600 mPa·s, even more preferably 4,500 to 12,000 mPa·s, most preferably 5,000 to 10,500 mPa·s and in particular 5,500 to 7,500 mPa·s or 7,500 to 10,000 mPa·s, measured in a 1 wt.-% aqueous solution.

The polyalkylene oxide may comprise a single polyalkylene oxide having a particular average molecular weight, or a mixture (blend) of different polymers, such as two, three, four or five polymers, e.g., polymers of the same chemical nature but different average molecular weight, polymers of different chemical nature but same average molecular weight, or polymers of different chemical nature as well as different molecular weight.

For the purpose of specification, a polyalkylene glycol has a molecular weight of up to 20,000 g/mol whereas a polyalkylene oxide has a molecular weight of more than 20,000 g/mol. The weight average over all molecular weights of all polyalkylene oxides that are contained in the pharmaceutical dosage form is more than 200,000 g/mol. Thus, polyalkylene glycols, if any, are preferably not taken into consideration when determining the weight average molecular weight of polyalkylene oxide.

For the purpose of specification, the term "zinc component" is meant to include elemental zinc as well as any kind of zinc containing component, e.g. inorganic or organic salts, complexes, alloys, oxides, chelates and organozinc compounds. Furthermore, the term "zinc component" comprises polymers which contain zinc, i.e. covalently bound, in ionic form or intercalated in form of a further zinc containing component. The zinc component may be or may comprise an organo-zinc compound of the general formula R₂Zn in which preferably R respectively independently stands for -C₁₋₈-aliphatic, -C₃₋₁₂-cycloaliphatic, -aryl, -heteroaryl, -C₁₋₈-aliphatic-C₃₋₁₂-cycloaliphatic, -C₁₋₈-aliphatic-aryl, -C₁₋₈-aliphatic-heteroaryl, -C₃₋₈-cycloaliphatic-C₁₋₈-aliphatic, -C₃₋₈-cycloaliphatic-aryl or -C₃₋₈-cycloaliphatic-heteroaryl; wherein "aliphatic" respectively is a branched or unbranched, saturated or a mono- or polyunsaturated, unsubstituted or mono- or polysubstituted, aliphatic hydrocarbon residue; "cycloaliphatic" respectively is a saturated or a mono- or polyunsaturated, unsubstituted or mono- or polysubstituted, alicyclic, mono- or multicyclic hydrocarbon residue; wherein with respect to "aliphatic" and "cycloaliphatic", "mono- or polysubstituted" means the mono- or polysubstitution of one or more hydrogen atoms by substituents selected independently of one another from the group consisting of -F, -Cl, -Br, -I, -CN, -NO₂, -CHO, -O, -R, -C(=O)R, -C(=O)H, -C(=O)OH, -C(=O)OR, -C(=O)NH₂, -C(=O)NHR, -C(=O)N(R)₂, -OH, -OR, -OC(=O)H, -OC(=O)R, -OC(=O)OR, -OC(=O)NHR, -OC(=O)NR₂, -SH, -SR, -SO₃H, -S(=O)₁₋₂-R, -S(=O)₁₋₂NH₂, -NH₂, -NHR, -NR₂, -N⁺R₃, -N⁺(R)₂O⁻, -NHC(=O)R, -NHC(=O)OR, -NHC(=O)NH₂, -NHC(=O)-NHR, -NH-C(=O)NR₂, -SiR₃ and -PO(OR)₂ with each "R" as defined above; "aryl", respectively independently, stands for a carbocyclic ring system with at least one aromatic ring, but without heteroatoms in this ring, wherein, optionally, the aryl residues can be condensed with further saturated, (partially) unsaturated or aromatic ring systems, and each aryl residue can be present in unsubstituted or mono- or polysubstituted form, wherein the aryl substituents can be the same or different and in any desired and possible position of the aryl; "heteroaryl" stands for a 5-, 6- or 7-membered cyclic aromatic residue, which contains 1, 2, 3, 4 or 5 heteroatoms, wherein the heteroatoms, the same or different, are nitrogen, oxygen or sulphur, and the heterocycle can be unsubstituted or mono-or polysubstituted; wherein in the case of the substitution on the heterocycle the substituents can be the same or different and can be in any desired and possible position of the heteroaryl; and wherein the heterocycle can also be part of a bi- or polycyclic system; wherein with respect to "aryl" and "heteroaryl", "mono- or polysubstituted" means the mono-or polysubstitution of one or more hydrogen atoms of the ring system by substituents selected from the group consisting of -F, -Cl, -Br, -I, -CN, -NO₂, -CHO, -O, -R, -C(=O)R, -C(=O)H, -C(=O)OH, -C(=O)OR, -C(=O)NH₂, -C(=O)NHR, -C(=O)-NR₂, -OH, -O(CH₂)₁₋₂O-, -OR, -OC(=O)H, -OC(=O)R, -OC(=O)OR, -OC(=O)NHR, -OC(=O)NR₂, -SH, -SR, -SO₃H, -S(=O)₁₋₂-R, -S(=O)₁₋₂NH₂, -NH₂, -NHR, -NR₂, -N⁺R₃, -N⁺R₂O⁻, -NHC(=O)R, -NHC(=O)OR, -NH-C(=O)NH₂, -NHC(=O)NHR, -NHC(=O)NR₂, -SiR₃ or -PO(OR)₂ with each "R" as defined above; wherein any N-ring atoms present can be respectively oxidized.

More preferably, R respectively independently stands for (i) an alkyl group containing from 1 to about 8 carbon atoms, preferably 1 to 6 carbon atoms, and most preferably 2 or 3 carbon atoms, or (ii) phenyl or naphthyl, or alkyl-substituted phenyl or naphthyl groups in which the alkyl groups contain from 1 to about 3 carbon atoms, or (iii) cycloalkyl groups containing from 4 to 6 ring carbon atoms; or (iv) the dicyclopentadienyl group; or (v) an alkoxide group containing from 1 to about 8 carbon atoms. Examples include, but are not limited to zinc carboxylates (e.g. zinc glutarate, zinc adipate, zinc isophthalate, zinc propionate), zinc alkyl, zinc alkoxide of monohydric and/or polyhydric alcohols, zinc cycloalkyl, zinc aryl or dicyclopentadienyl compounds (e.g. dimethylzinc, diethylzinc, dipropylzinc, di-isopropylzinc, dibutylzinc, di-isobutylzinc, di-t-butylzinc, dipentylzinc salts, dihexyl-and diheptyl- and dioctylzinc salts, di-2-ethylhexylzinc, diphenylzinc, ditolylzinc, dicyclobutylzinc, dicyclopentylzinc, di-methylcyclopentylzinc, dicyclohexylzinc, methyl phenylzinc, methyl tolylzinc, methyl naphthylzinc, ethyl phenylzinc), zinc salts of a strong acid (e.g. zinc nitrate) or an organic acid (e.g. zinc acetylacetonate, zinc acetate, zinc salts of fatty acids), zinc phthalocyanines, zinc halides, organic zinc halides (e.g. alkyl or aryl zinc halides), zinc naphthalocyanines, zinc porphyrins, zinc (meth)acrylates (e.g. zinc acrylate, zinc diacrylate, zinc methacrylate, zinc dimethacrylate), halogenated thiophenol zinc salts (e.g. zinc salt of pentachlorothiophenol), and mixtures thereof. Generally, the polyalkylene oxide is obtainable by polymerizing alkylene oxide in the presence of the zinc component of the general formula R₂Zn. Particularly preferably, the polyalkylene oxide is obtainable by polymerizing alkylene oxide in the presence of the zinc component, wherein the zinc component is a zinc alkoxide of monohydric and/or polyhydric alcohols.

In another preferred aspect, the polyalkylene oxide is obtainable by polymerizing alkylene oxide in the presence of the zinc component, wherein the amount of the zinc component is in the range of from 0.01 to 1 mol-% based on the zinc atom content per mol of the alkylene oxide monomers.

If the polyalkylene oxide is obtained by polymerizing alkylene oxide in the presence of the zinc component, the zinc component is preferably present in a deactivated form after the polymerization reaction, most preferably in a hydrolyzed form and even more preferably as Zn(OH)₂, Zn(OH)₄²⁻, ZnO or mixtures thereof. Generally, the zinc component comprises substantially no zinc stearate and/or zinc sulfate. In a particularly preferred aspect, the pharmaceutical dosage form according to the invention comprises substantially no zinc stearate and/or zinc sulfate.

For the purpose of specification, unless expressly stated otherwise, "substantially no" is preferably to be regarded as below 1 ppm relative to the total weight of the dosage form, more preferably below 0.1 ppm relative to the total weight of the dosage form, still more preferably "not detectable", i.e. below the detection limit. Preferably, the zinc component is or comprises the reaction product of a dihydrocarbyl zinc compound and a linear alkanediol such as 1,4-butanediol. Such reaction products are useful as catalysts in the polymerization of cyclic oxides such as ethylene oxide and are known for example from US 4,667,01 3. In another preferred aspect, the zinc component is or comprises the reaction product of a dihydrocarbyl zinc compound and an emulsion of a polyol-surfactant-dispersion aid. Such reaction products are useful as catalysts in the polymerization of alkylene oxides and are known for example from EP 0 239 973. Preferably, the zinc component is obtainable by reacting
- a dialkyl zinc (e.g. dimethyl zinc, diethyl zinc, dipropyl zinc or dibutyl zinc), diphenyl zinc or dicyclobutyl zinc with
- an aliphatic polyhydric alcohol (e.g. ethylene glycol, propylene glycol, 1,2-butanediol, 1,3-butanediol, 1,4-butanediol, 2,3-butanediol, 1,5-pentanediol, 2,3,4-pentanetriol, glycerol or pentaerythritol), and
- a monohydric alcohol (e.g. methanol, ethanol, propanol, butanol or pentanol).
The zinc component is preferably obtainable as is described in US 5,326,852 and US 6,979,722. The zinc component may be the remainder of the polymerization catalyst that was used in the course of the polymerization of the polyalkylene oxide when the polyalkylene oxide was manufactured, wherein preferably the amount of the zinc component is in the range of from 0.01 to 1 mol-% based on the zinc atom content per mol of the alkylene oxide units contained in the polyalkylene oxide. According to this aspect, after the polymerization reaction, the remainder of the catalyst is preferably present in a deactivated form, preferably in a hydrolyzed form, more preferably as Zn(OH)₂, Zn(OH)₄²⁻, ZnO or mixtures thereof.

In a preferred embodiment, the polyalkylene oxide contained in the pharmaceutical dosage form according to the invention is obtainable by polymerizing alkylene oxide in presence of the zinc component, preferably of the zinc component according to US 4,667,013 or US 5,326,852 or US 6,979,722 or EP 0 239 973 as described above. In a particularly preferred embodiment, the polyalkylene oxide contained in the pharmaceutical dosage form according to the invention is obtainable by polymerizing alkylene oxide in presence of the zinc component, which is preferably obtainable by reacting a dialkyl zinc with an aliphatic polyhydric alcohol and a monohydric alcohol, as disclosed in US 5,326,852 or US 6,979,722, wherein preferably the amount of the zinc component is in the range of from 0.01 to 1 mol-% based on the zinc atom content per mol of the alkylene oxide monomers. The zinc component may be the constituent of a polyalkylene oxide composition also comprising the polyalkylene oxide, wherein an aqueous dispersion of the pure polyalkylene oxide composition has a lower pH value than a dispersion of an otherwise comparable polyalkylene oxide not containing the zinc component, typically containing substantially no zinc.

Preferably, the pH value is measured several days after dispersing the pure polyalkylene oxide composition in water, preferably after four days.

In a preferred embodiment, an aqueous dispersion of the pure polyalkylene oxide composition in pure water at 25°C and at a concentration of 1 wt.-% has a pH value of at most 7.7.

Preferably, an aqueous dispersion of the pure polyalkylene oxide composition in pure water at 25°C and at a concentration of 1 wt.-% after several days, preferably after four days, has a pH value of at most 7.7. Preferably, an aqueous dispersion of the pure polyalkylene oxide composition in pure water at 25°C and at a concentration of 1 wt.-% after several days, preferably after four days, has a pH value of at most 7.6, more preferably at most 7.5, still more preferably at most 7.4, yet more preferably at most 7.3, even more preferably at most 7.2, most preferably at most 7.1 and in particular at most 7.0. In still another preferred embodiment, an aqueous dispersion of the pure polyalkylene oxide composition in pure water at 25°C and at a concentration of 1 wt.-% after several days, preferably after four days, has a pH value of at most 6.9, more preferably at most 6.8 and most preferably at most 6.7. The content of the zinc component in the pharmaceutical dosage form according to the invention is at least 1 ppm, more preferably at least 2 ppm, still more preferably at least 5 ppm, yet more preferably at least 7 ppm, even more preferably at least 10 ppm, most preferably at least 15 ppm, and in particular at least at least 20 ppm, relative to the total weight of the pharmaceutical dosage form.

Preferably, the content of the zinc component in the pharmaceutical dosage form is below 0.01 g, more preferably below 8 mg and most preferably below 5 mg.
Preferably, the content of the zinc component in the pharmaceutical dosage form according to the invention is at most 10,000 ppm, more preferably at most 8,000 ppm, still more preferably at most 6,000 ppm, yet more preferably at most 5,000 ppm, even more preferably at most 4,000 ppm, most preferably at most 3,000 ppm, and in particular at most 2,000 ppm, relative to the total weight of the pharmaceutical dosage form.

Preferably, the content of the zinc component in the pharmaceutical dosage form is at most 1,000 ppm, more preferably at most 950 ppm, still more preferably at most 900 ppm, yet more preferably at most 850 ppm, even more preferably at most 800 ppm, most preferably at most 750 ppm, and in particular at most 700 ppm, relative to the total weight of the pharmaceutical dosage form.

In another preferred aspect, the content of the zinc component in the pharmaceutical dosage form according to the invention is preferably at most 600 ppm, more preferably at most 400 ppm, still more preferably at most 200 ppm, most preferably at most 100 ppm and in particular at most 80 ppm, relative to the total weight of the pharmaceutical dosage form.

Preferably, the content of the zinc component in the pharmaceutical dosage form is below 200 ppm, more preferably below 200 ppm.

For the purpose of specification, unless expressly stated otherwise, "ppm" is to be regarded as ppmw, i.e. parts per million by weight, so that 1 ppm corresponds to 0.0001 wt.-%.

In a preferred aspect, the pharmaceutical dosage form according to the invention contains a polyalkylene oxide composition that comprises the polyalkylene oxide and the zinc component. Preferably, the content of said zinc component is at least 1 ppm, more preferably at least 2 ppm, still more preferably at least 5 ppm, yet more preferably at least 7 ppm, even more preferably at least 10 ppm, most preferably at least 15 ppm, and in particular at least 20 ppm, relative to the total weight of the polyalkylene oxide composition. Further preferably the content of said zinc component is at least 50 ppm, more preferably at least 100 ppm, still more preferably at least 200 ppm, yet more preferably at least 400 ppm, even more preferably at least 600 ppm, most preferably at least 700 ppm, and in particular at least 800 ppm, relative to the total weight of the polyalkylene oxide composition. The content of the zinc component in the pharmaceutical dosage form according to the invention may be in the range of from 0.01 to 1 mol-%, more preferably 0.015 to 0.5 mol-%, most preferably 0.015 to 0.1 mol-% and in particular 0.018 to 0.05 mol-% based on the zinc atom content per mol of the alkylene oxide units which are contained in the polyalkylene oxide.

In another preferred aspect, the content of the zinc component in the pharmaceutical dosage form amounts to 860±700 ppm, more preferably 860±600 ppm, still more preferably 860±500 ppm, yet more preferably 860±400 ppm, even more preferably 860±300 ppm, most preferably 860±200 ppm and in particular 860±100 ppm, relative to the total weight of the polyalkylene oxide composition.

Preferably, the content of the zinc component in the pharmaceutical dosage form is at most 20,000 ppm, more preferably at most 18,000 ppm, most preferably at most 15,000 ppm and in particular at most 12,000 ppm, relative to the total weight of the polyalkylene oxide composition. Preferably, the content of the zinc component in the pharmaceutical dosage form is at most 1,000 ppm, more preferably at most 950 ppm, still more preferably at most 900 ppm, yet more preferably at most 850 ppm, even more preferably at most 800 ppm, most preferably at most 750 ppm, and in particular at most 700 ppm, relative to the total weight of the polyalkylene oxide composition. Preferably, the content of the zinc component in the pharmaceutical dosage form is at most 600 ppm, more preferably at most 500 ppm, still more preferably at most 400 ppm, yet more preferably at most 300 ppm, most preferably at most 250 ppm and in particular at most 200 ppm, relative to the total weight of the polyalkylene oxide composition.

Preferably, the zinc component is contained in an amount which does not have any physiological effect, in particular adverse effects such as causing emesis. The zinc component, preferably zinc sulfate, is preferably contained in an amount of less than 0.01 g, more preferably less than 1 mg, still more preferably less than 0.1 mg, even more preferably less than 0.01 mg, yet more preferably less than 1 µg, most preferably less than 0.1 µg and in particular less than 0.01 µg. The zinc component, preferably zinc sulfate, is preferably contained in the pharmaceutical dosage form according to the invention in an amount of less than 10,000 ppm, more preferably less than 1,000 ppm, most preferably less than 500 ppm and in particular less than 100 ppm.

In a preferred embodiment, the zinc component is homogeneously distributed in the pharmaceutical dosage form according to the invention. Preferably, the polyalkylene oxide or the polyalkylene oxide composition and the zinc component are intimately homogeneously distributed in the pharmaceutical dosage form so that the pharmaceutical dosage form does not contain any segments where either zinc component is present in the absence of polyalkylene oxide and polyalkylene oxide composition, respectively, or where the polyalkylene oxide and the polyalkylene oxide composition, respectively, is present in the absence of the zinc component.

When the pharmaceutical dosage form is film coated, the zinc component is preferably homogeneously distributed in the core of the pharmaceutical dosage form, i.e. the film coating preferably does not contain a zinc component. The polyalkylene oxide composition provides an enhanced shelf life to the pharmaceutical dosage form according to the invention. The content of elemental zinc, either in cationic or neutral form, is at least 1 ppm, more preferably at least 2 ppm, still more preferably at least 5 ppm, yet more preferably at least 7 ppm, even more preferably at least 10 ppm, most preferably at least 15 ppm, and in particular at least 20 ppm, relative to the total weight of the pharmaceutical dosage form. In another preferred aspect, the content of elemental zinc, either in cationic or neutral form, in the pharmaceutical dosage form according to the invention is at least 25 ppm, more preferably at least 35 ppm, still more preferably at least 45 ppm, yet more preferably at least 55 ppm, even more preferably at least 65 ppm, most preferably at least 75 ppm, and in particular at least 85 ppm, relative to the total weight of the pharmaceutical dosage form. Preferably, the content of elemental zinc, either in cationic or neutral form, in the pharmaceutical dosage form according to the invention is at least 100 ppm, more preferably at least 300 ppm, still more preferably at least 500 ppm, yet more preferably at least 800 ppm, even more preferably at least 1,000 ppm, most preferably at least 3,000 ppm, and in particular at least 5,000 or 8,000 ppm, relative to the total weight of the pharmaceutical dosage form.

Particularly preferably, the content of elemental zinc, either in cationic or neutral form, is in the range of from 200 ppm to 800 ppm. The content of elemental zinc, either in cationic or neutral form, is 250±200 ppm, more preferably 250±150 ppm, still more preferably 250±130 ppm, even more preferably 250±110 ppm, yet more preferably 250±90 ppm, most preferably 250±70 ppm and in particular 250±50 ppm, relative to the total weight of the pharmaceutical dosage form. In another preferred aspect, the content of elemental zinc, either in cationic or neutral form, in the pharmaceutical dosage form according to the invention is 300±200 ppm, more preferably 300±150 ppm, still more preferably 300±130 ppm, even more preferably 300±110 ppm, yet more preferably 300±90 ppm, most preferably 300±70 ppm and in particular 300±50 ppm, relative to the total weight of the pharmaceutical dosage form. In still another preferred aspect, the content of elemental zinc, either in cationic or neutral form, is 500±400 ppm, more preferably 500±300 ppm, still more preferably 500±250 ppm, even more preferably 500±200 ppm, yet more preferably 500±150 ppm, most preferably 500±100 ppm and in particular 500±50 ppm, relative to the total weight of the pharmaceutical dosage form. In yet another preferred aspect, the content of elemental zinc, either in cationic or neutral form, is 700±600 ppm, more preferably 700±500 ppm, still more preferably 700±400 ppm, even more preferably 700±300 ppm, yet more preferably 700±200 ppm, most preferably 700±100 ppm and in particular 700±50 ppm, relative to the total weight of the pharmaceutical dosage form.

Preferably, the content of elemental zinc, either in cationic or neutral form, in the pharmaceutical dosage form according to the invention is at most 1.0 wt.-% (10,000 ppm), most preferably at most 0.8 wt.-% relative to the total weight of the pharmaceutical dosage form. Preferably, the content of elemental zinc, either in cationic or neutral form, in the pharmaceutical dosage form according to the invention is at most 1,000 ppm, more preferably at most 950 ppm, still more preferably at most 900 ppm, yet more preferably at most 850 ppm, even more preferably at most 800 ppm, most preferably at most 750 ppm, and in particular at most 700 ppm, relative to the total weight of the pharmaceutical dosage form.

Preferably, the content of elemental zinc, either in cationic or neutral form, preferably zinc (II), in the pharmaceutical dosage form is in the range of from 0.01 to 1 mol-% based on the zinc atom content per mol of the alkylene oxide units contained in the polyalkylene oxide. Preferably, the content of zinc (II) in the pharmaceutical dosage form according to the invention is at least 0.01 mol-%, more preferably at least 0.03 mol-%, still more preferably at least 0.06 mol-%, yet more preferably at least 0.10 mol-%, even more preferably at least 0.15 mol-%, most preferably at least 0.20 mol-%, and in particular at least 0.5 mol-%, relative to the amount of substance of the pharmacologically active ingredient. In another preferred aspect, the content of zinc (II) in the pharmaceutical dosage form according to the invention is at least 1 mol-%, more preferably at least 4 mol-%, still more preferably at least 8 mol-%, yet more preferably at least 12 mol-%, even more preferably at least 15 mol-%, most preferably at least 18 mol-%, and in particular at least 20 mol-%, relative to the amount of substance of the pharmacologically active ingredient.

Preferably, the content of zinc (II) in the pharmaceutical dosage form is at most 50 mol-%, more preferably at most 45 mol-%, still more preferably at most 40 mol-%, yet more preferably at most 35 mol-%, even more preferably at most 30 mol-%, most preferably at most 25 mol-%, and in particular at most 20 mol-%, relative to the amount of substance of the pharmacologically active ingredient. Preferably, the content of zinc (II) in the pharmaceutical dosage form is at least 1·10⁻⁷ mol, more preferably at least 2·10⁻⁷ mol, still more preferably at least 3·10⁻⁷ mol, yet more preferably at least 4·10⁻⁷ mol, even more preferably at least 5·10⁻⁷ mol, most preferably at least 7·10⁻⁷ mol, and in particular at least 1·10⁻⁶ mol. In another preferred aspect, the content of zinc (II) in the pharmaceutical dosage form according to the invention is at least 1·10⁻⁵ mol, more preferably at least 1·10⁻⁴ mol, still more preferably at least 1·10⁻³ mol, yet more preferably at least 5·10⁻³ mol, even more preferably at least 8·10⁻³ mol, most preferably at least 9·10⁻³ mol, and in particular at least 1·10⁻² mol.

Preferably, the content of zinc (II) is at most 100·10⁻³ mol, more preferably at most 80·10⁻³ mol, still more preferably at most 70·10⁻³ mol, yet more preferably at most 60·10⁻³ mol, even more preferably at most 50·10⁻³ mol, most preferably at most 40·10⁻³ mol, and in particular at most 35·10⁻³ mol. Preferably, the content of zinc (II) in the pharmaceutical dosage form according to the invention is at most 1·10⁻³ mol, more preferably at most 7·10⁻⁴ mol, still more preferably at most 5·10⁻⁴ mol, yet more preferably at most 4·10⁻⁴ mol, even more preferably at most 3·10⁻⁴ mol, most preferably at most 2·10⁻⁴ mol, and in particular at most 1·10⁻⁴ mol.

Preferably, the content of the polyalkylene oxide or of the polyalkylene oxide composition is at least 10 wt.-%, more preferably at least 15 wt.-%, still more preferably at least 20 wt.-%, even more preferably at least 25 wt.-%, most preferably at least 30 wt.-% and in particular at least 35 wt.-% based on the total weight of the pharmaceutical dosage form, wherein the content of the zinc component is in the range of from 0.01 to 1 mol-% based on the zinc atom content per mol of the alkylene oxide units contained in the polyalkylene oxide.

Preferably, the content of the polyalkylene oxide or of the polyalkylene oxide composition is within the range of from 20 to 99 wt.-%, more preferably 25 to 95 wt.-%, still more preferably 30 to 90 wt.-%, yet more preferably 30 to 85 wt.-%, most preferably 30 to 80 wt.-% and in particular 30 to 75 wt.-% or 45 to 70 wt.-%, based on the total weight of the pharmaceutical dosage form. The content of the polyalkylene oxide or of the polyalkylene oxide composition is at least 20 wt.-%, preferably at least 25 wt.-%, more preferably at least 30 wt.-%, still more preferably at least 35 wt.-% and in particular at least 40 wt.-%, based on the total weight of the pharmaceutical dosage form. The overall content of the polyalkylene oxide or of the polyalkylene oxide composition is within the range of 25±5 wt.-%. In another preferred aspect, the overall content of the polyalkylene oxide or of the polyalkylene oxide composition is within the range of 35±15 wt.-%, more preferably 35±10 wt.-%, and in particular 35±5 wt.-%. Preferably, the overall content of the polyalkylene oxide or of the polyalkylene oxide composition is within the range of 45±20 wt.-%, more preferably 45±15 wt.-%, most preferably 45±10 wt.-%, and in particular 45±5 wt.-%. In yet another preferred aspect, the overall content of the polyalkylene oxide or of the polyalkylene oxide composition is within the range of 55±20 wt.-%, more preferably 55±15 wt.-%, most preferably 55±10 wt.-%, and in particular 55±5 wt.-%. In a further preferred aspect, the overall content of the polyalkylene oxide or of the polyalkylene oxide composition is within the range of 65±20 wt.-%, more preferably 65±15 wt.-%, most preferably 65±10 wt.-%, and in particular 65±5 wt.-%. In still a further aspect, the overall content of the polyalkylene oxide or of the polyalkylene oxide composition is within the range of 75±20 wt.-%, more preferably 75±15 wt.-%, most preferably 75±10 wt.-%, and in particular 75±5 wt.-%. In yet a further a preferred aspect, the overall content of the polyalkylene oxide or of the polyalkylene oxide composition is within the range of 80±15 wt.-%, more preferably 80±10 wt.-%, and most preferably 80±5 wt.-%.

Preferably, the pharmacologically active ingredient and the polyalkylene oxide or the polyalkylene oxide composition are intimately homogeneously distributed in the pharmaceutical dosage form so that the pharmaceutical dosage form does not contain any segments where either pharmacologically active ingredient is present in the absence of polyalkylene oxide and polyalkylene oxide composition, respectively, or where the polyalkylene oxide and the polyalkylene oxide composition, respectively, is present in the absence of the pharmacologically active ingredient.

When the pharmaceutical dosage form is film coated, the polyalkylene oxide or the polyalkylene oxide composition is preferably homogeneously distributed in the core of the pharmaceutical dosage form, i.e. the film coating preferably does not contain polyalkylene oxide or polyalkylene oxide composition. Nonetheless, the film coating as such may of course contain one or more polymers, which however, preferably differ from the polyalkylene oxide or polyalkylene oxide composition contained in the core.

The polyalkylene oxide may be combined with or the polyalkylene oxide composition may additionally comprise one or more different polymers selected from the group consisting of polyalkylene oxide, preferably polymethylene oxide, polyethylene oxide, polypropylene oxide; polyethylene, polypropylene, polyvinyl chloride, polycarbonate, polystyrene, polyvinylpyrrolidone, poly(alk)acrylate, poly(hydroxy fatty acids), such as for example poly(3-hydroxybutyrate-co-3-hydroxyvalerate) (Biopol^{®}), poly(hydroxyvaleric acid); polycaprolactone, polyvinyl alcohol, polyesteramide, polyethylene succinate, polylactone, polyglycolide, polyurethane, polyamide, polylactide, polyacetal (for example polysaccharides optionally with modified side chains), hydroxypropyl methylcellulose (Hypromellose); polylactide/glycolide, polylactone, polyglycolide, polyorthoester, polyanhydride, block polymers of polyethylene glycol and polybutylene terephthalate (Polyactive^{®}), polyanhydride (Polifeprosan), copolymers thereof, block-copolymers thereof, and mixtures of at least two of the stated polymers, or other polymers with the above characteristics.

Preferably, the relative weight ratio of the polyalkylene oxide or polyalkylene oxide composition to the pharmacologically active ingredient is at least 0.5:1, more preferably at least 1:1, at least 2:1, at least 3:1, at least 4:1, at least 5:1, at least 6:1, at least 7:1, at least 8:1 or at least 9:1; still more preferably at least 10:1 or at least 15:1, yet more preferably at least 20:1, most preferably at least 30:1 and in particular at least 40:1. In a preferred embodiment, the relative weight ratio of the polyalkylene oxide or polyalkylene oxide composition to the pharmacologically active ingredient is within the range of from 3:1 to 50:1, more preferably 3:1 to 40:1 and in particular 3:1 to 30:1. Generally, the polyalkylene oxide composition according to the invention provides an enhanced stability to the pharmacologically active ingredient.

Besides the pharmacologically active ingredient and the polyalkylene oxide or the polyalkylene oxide composition, the pharmaceutical dosage form according to the invention may contain further ingredients, e.g. one or more conventional pharmaceutical excipient(s), e.g. inorganic salts, fillers, glidants, binding agents, granulating agents, anti-caking agents, lubricants, flavours, dyes, and/or preservatives.

Preferably, the pharmaceutical dosage form does not contain a zinc component as a lubricant or glidant. More preferably, the pharmaceutical dosage form contains substantially no zinc stearate as a lubricant or glidant which is particularly used to obtain desired flow and less friction during compressing operation.

In a preferred aspect, the pharmaceutical dosage form according to the invention contains at most 1,000 ppm, more preferably at most 500 ppm, still more preferably at most 100 ppm, even more preferably at most 10 ppm, yet more preferably at most 1 ppm, most preferably at most 0.1 ppm and in particular at most 0.01 ppm of a calcium component, or contains substantially no calcium component.

Preferably, the pharmaceutical dosage form comprises a plasticizer. The plasticizer improves the processability of the polyalkylene oxide or polyalkylene oxide composition. A preferred plasticizer is polyalkylene glycol, like polyethylene glycol, triacetin, fatty acids, fatty acid esters, waxes and/or microcrystalline waxes. Particularly preferred plasticizers are polyethylene glycols, such as PEG 6000.

Preferably, the content of the plasticizer is within the range of from 0.1 to 25 wt.-%, more preferably 0.5 to 22.5 wt.-%, still more preferably 1.0 to 20 wt.-%, yet more preferably 2.5 to 17.5 wt.-%, most preferably 5.0 to 15 wt.-% and in particular 7.5 to 12.5 wt.-%, based on the total weight of the pharmaceutical dosage form. The plasticizer may be a polyalkylene glycol having a content within the range of 10±8 wt.-%, more preferably 10±6 wt.-%, still more preferably 10±5 wt.-%, yet more preferably 10±4 wt.-%, most preferably 10±3 wt.-%, and in particular 10±2 wt.-%, based on the total weight of the pharmaceutical dosage form. The plasticizer may be a polyalkylene glycol having a content within the range of 15±8 wt.-%, more preferably 15±6 wt.-%, still more preferably 15±5 wt.-%, yet more preferably 15±4 wt.-%, most preferably 15±3 wt.-%, and in particular 15±2 wt.-%, based on the total weight of the pharmaceutical dosage form.

In a preferred aspect, the relative weight ratio of the polyalkylene oxide or polyalkylene oxide composition to the optionally contained polyalkylene glycol is within the range of 4.2±2 : 1, more preferably 4.2±1.5 : 1, still more preferably 4.2±1 : 1, yet more preferably 4.2±0.5 : 1, most preferably 4.2±0.2 : 1, and in particular 4.2±0.1 : 1. This ratio satisfies the requirements of relative high polyalkylene oxide content and good extrudability.

When manufacturing the pharmaceutical dosage forms from slices that are obtained by cutting an extrudate strand, the weight of the slices determines the weight of the resulting dosage form. Pronounced variation in weight of these slices results in an accordant weight deviation of dosage forms from the target weight. The weight variation of slices depends strongly on the surface properties of the extrudate strand. A strand with a thoroughly smooth surface allows the generation of slices exhibiting a low weight variation. In contrast, a wavy or shark skinned strand results in slices exhibiting a higher weight variation thereby increasing the number of rejects. The surface properties of the extrudate strand can be triggered by the polyalkylene oxide: polyalkylene glycol weight ratio.

Preferably, the pharmaceutical dosage form further comprises an anti-oxidant. Suitable antioxidants include ascorbic acid, butylated hydroxyanisole (BHA), butylated hydroxytoluene (BHT), salts of ascorbic acid, monothioglycerol, phosphorous acid, vitamin C, vitamin E and the derivatives thereof, coniferyl benzoate, nordihydroguajaretic acid, gallus acid esters, sodium bisulfite, particularly preferably butylhydroxytoluene or butylhydroxyanisole and α-tocopherol. The antioxidant is preferably used in quantities of 0.01 to 10 wt.-%, preferably of 0.03 to 5 wt.-%, relative to the total weight of the pharmaceutical dosage form. The pharmaceutical dosage form may comprise an acid, preferably citric acid. The amount of acid is preferably in the range of 0.01 to about 20 wt.-%, more preferably in the range of 0.02 to about 10 wt.-%, and most preferably in the range of 0.05 to about 5 wt.-% relative to the total weight of the pharmaceutical dosage form. The pharmaceutical dosage form may contain a natural, semisynthetic or synthetic wax. Waxes with a softening point of at least 50 °C, more preferably 60 °C are preferred. Carnauba wax and beeswax are particularly preferred, especially carnauba wax. The pharmaceutical dosage form may further comprise another polymer which is preferably selected from cellulose esters and cellulose ethers, in particular hydroxypropyl methylcellulose (HPMC). The amount of the further polymer, preferably hydroxypropyl methylcellulose, preferably ranges from 0.1 wt.-% to about 30 wt.-%, more preferably in the range of 1.0 wt.-% to about 20 wt.-%, and most preferably in the range of 2.0 wt.-% to about 15 wt.-% relative to the total weight of the pharmaceutical dosage form.

The pharmaceutical dosage form is preferably an oral dosage form, particularly a tablet. It is also possible, however, to administer the pharmaceutical dosage form via different routes and thus, the pharmaceutical dosage form may alternatively be adapted for buccal, lingual, rectal or vaginal administration. Implants are also possible.

In a preferred embodiment, the pharmaceutical dosage form according to the invention is monolithic or multiparticulate. The pharmaceutical dosage may be a round tablet. The tablets preferably have a diameter in the range of about 1 mm to about 30 mm, in particular in the range of about 2 mm to about 25 mm, more in particular about 5 mm to about 23 mm, even more in particular about 7 mm to about 13 mm; and a thickness in the range of about 1.0 mm to about 12 mm, in particular in the range of about 2.0 mm to about 10 mm, even more in particular from 3.0 mm to about 9.0 mm, even further in particular from about 4.0 mm to about 8.0 mm.

In another preferred aspect, the pharmaceutical dosage form is an oblong tablet. Tablets of this embodiment preferably have a lengthwise extension (longitudinal extension) of about 1 mm to about 30 mm, in particular in the range of about 2 mm to about 25 mm, more in particular about 5 mm to about 23 mm, even more in particular about 7 mm to about 20 mm; and a thickness in the range of about 1.0 mm to about 12 mm, in particular in the range of about 2.0 mm to about 10 mm, even more in particular from 3.0 mm to about 9.0 mm, even further in particular from about 4.0 mm to about 8.0 mm.

The pharmaceutical dosage form has preferably a weight in the range of 0.01 to 1.5 g, more preferably in the range of 0.05 to 1.2 g, still more preferably in the range of 0.1 g to 1.0 g, yet more preferably in the range of 0.2 g to 0.9 g, and most preferably in the range of 0.25 g to 0.8 g.

The pharmaceutical dosage form according to the invention may be prepared by thermoforming.

Preferably, the pharmaceutical dosage form according to the invention is prepared by hot-melt extrusion, although also other methods of thermoforming may be used in order to manufacture the pharmaceutical dosage form according to the invention such as press-molding at elevated temperature or heating of tablets that were manufactured by conventional compression in a first step and then heated above the softening temperature of the polymer in the tablet in a second step to form hard tablets. In this regards, thermoforming means the forming or molding of a mass after the application of heat. In a preferred embodiment, the pharmaceutical dosage form is thermoformed by hot-melt extrusion.

In a preferred embodiment, the pharmaceutical dosage form is prepared by hot melt-extrusion, preferably by means of a twin-screw-extruder. Melt extrusion preferably provides a melt-extruded strand that is preferably cut into monoliths, which are then compressed and formed into tablets. In this regard, the term "tablets" is preferably not to be understood as dosage forms being made by compression of powder or granules (*compressi*) but rather, as shaped extrudates. Preferably, compression is achieved by means of a die and a punch, preferably from a monolithic mass obtained by melt extrusion. If obtained via melt extrusion, the compressing step is preferably carried out with a monolithic mass exhibiting ambient temperature, that is, a temperature in the range from 20 to 25 °C. The strands obtained by way of extrusion can either be subjected to the compression step as such or can be cut prior to the compression step. This cutting can be performed by usual techniques, for example using rotating knives or compressed air. Alternatively, the shaping can take place as described in EP-A 240 906 by the extrudate being passed between two counter-rotating calender rolls and being shaped directly to tablets. It is of course also possible to subject the extruded strands to the compression step or to the cutting step when still warm, that is more or less immediately after the extrusion step. The extrusion is preferably carried out by means of a twin-screw extruder.

The pharmaceutical dosage form of the invention can optionally be provided, partially or completely, with a conventional coating. The pharmaceutical dosage forms of the present invention are preferably film coated with conventional film coating compositions. Particularly preferably, the pharmaceutical dosage forms according to the invention are either not coated at all or completely coated, but preferably not partially coated.

Suitable coating materials are commercially available, e.g. under the trademarks Opadry^{®} and Eudragit^{®}.

Examples of suitable materials include cellulose esters and cellulose ethers, such as methylcellulose (MC), hydroxypropylmethylcellulose (HPMC), hydroxypropylcellulose (HPC), hydroxyethylcellulose (HEC), sodium carboxymethylcellulose (Na-CMC), ethylcellulose (EC), cellulose acetate phthalate (CAP), hydroxypropylmethylcellulose phthalate (HPMCP); poly(meth)acrylates, such as aminoalkylmethacrylate copolymers, ethylacrylate methylmethacrylate copolymers, methacrylic acid methylmethacrylate copolymers, methacrylic acid methylmethacrylate copolymers; vinyl polymers, such as polyvinylpyrrolidone, polyvinyl-acetatephthalate, polyvinyl alcohol, polyvinylacetate; and natural film formers, such as shellack. Preferably, the coating is water-soluble. It may be based on polyvinyl alcohol, such as polyvinyl alcohol-part. Hydrolyzed, and may additionally contain polyethylene glycol, such as macrogol 3350, and/or pigments. In another preferred embodiment, the coating is based on hydroxypropylmethylcellulose, preferably hypromellose type 2910 having a viscosity of 3 to 15 mPa·s.

The coating can be resistant to gastric juices and dissolve as a function of the pH value of the release environment. By means of this coating, it is possible to ensure that the pharmaceutical dosage form according to the invention passes through the stomach undissolved and the pharmacologically active ingredient is only released in the intestines. The coating which is resistant to gastric juices preferably dissolves at a pH value of between 5 and 7.5. Corresponding materials and methods for the delayed release of pharmacologically active ingredients and for the application of coatings which are resistant to gastric juices are known to the person skilled in the art, for example from "Coated Pharmaceutical dosage forms - Fundamentals, Manufacturing Techniques, Biopharmaceutical Aspects, Test Methods and Raw Materials" by Kurt H. Bauer, K. Lehmann, Hermann P. Osterwald, Rothgang, Gerhart, 1 st edition, 1998, Medpharm Scientific Publishers.

The coating can also be applied e.g. to improve the aesthetic impression and/or the taste of the pharmaceutical dosage forms and the ease with which they can be swallowed. Coating the pharmaceutical dosage forms of the present invention can also serve other purposes, e.g. improving stability and shelf-life. Suitable coating formulations comprise a film forming polymer such as, for example, polyvinyl alcohol or hydroxypropyl methylcellulose, e.g. hypromellose, a plasticizer such as, for example, a glycol, e.g. propylene glycol or polyethylene glycol, an opacifier, such as, for example, titanium dioxide, and a film smoothener, such as, for example, talc. Suitable coating solvents are water as well as organic solvents. Examples of organic solvents are alcohols, e.g. ethanol or isopropanol, ketones, e.g. acetone, or halogenated hydrocarbons, e.g. methylene chloride. Optionally, the coating can contain a therapeutically effective amount of one or more pharmacologically active ingredients to provide for an immediate release of said pharmacologically active ingredient and thus for an immediate relief of the symptoms treated by said pharmacologically active ingredient. Coated dosage forms of the present invention are preferably prepared by first making the cores and subsequently coating said cores using conventional techniques, such as coating in a coating pan.

In one preferred embodiment, the pharmaceutical dosage form according to the invention contains the pharmacologically active ingredient in a controlled-release matrix that provides controlled release of the pharmacologically active ingredient contained therein.

The controlled release of the pharmaceutical dosage form according to the invention preferably does not rely on a coating that remains intact during the release phase and covers the matrix composition in such a manner that only a specific surface area is subject to erosion. Thus, the surface area of the pharmaceutical dosage form according to the invention from which the active substance is released is preferably not kept substantially constant by means of such a coating. On the contrary, the controlled release of the pharmaceutical dosage form according to the invention is preferably based on the properties of the matrix in which the pharmacologically active ingredient is embedded so that inert coatings can be completely omitted. Thus, while the pharmaceutical dosage form according to the invention may be coated with conventional coating materials such as polyvinyl alcohol, it is preferably not coated with inert coating materials that serve the purpose of permanently covering a substantial portion of the outer surface of the pharmaceutical dosage form in order to allow drug release only through a predetermined, uncoated portion. Thus, in a preferred embodiment, the pharmaceutical dosage form according to the invention is uncoated, or it is coated with a coating material that substantially covers the complete outer surface of the pharmaceutical dosage form, but does not leave a certain portion uncoated.

In a preferred embodiment, the pharmacologically active ingredient is embedded in a controlled-release matrix comprising the polyalkylene oxide and the zinc component, preferably of the polyalkylene oxide composition that is composed of the polyalkylene oxide and the zinc component.

Controlled release of an pharmacologically active ingredient from an oral dosage form is known to a person skilled in the art. For the purpose of specification, controlled release encompasses delayed release, retarded release, sustained release, extended release, prolonged release, and the like.

Controlled or prolonged release is understood according to the invention preferably to mean a release profile in which the pharmacologically active ingredient is released over a relatively long period with reduced intake frequency with the purpose of extended therapeutic action. Preferably, the meaning of the term "prolonged release" is in accordance with the European guideline on the nomenclature of the release profile of pharmaceutical dosage forms (CHMP). This is achieved in particular with peroral administration. The expression "at least partially delayed or prolonged release" covers according to the invention any pharmaceutical dosage forms which ensure modified release of the pharmacologically active ingredient contained therein. The pharmaceutical dosage forms preferably comprise coated or uncoated pharmaceutical dosage forms, which are produced with specific auxiliary substances, by particular processes or by a combination of the two possible options in order to purposefully change the release rate or location of release.

In the case of the pharmaceutical dosage forms according to the invention, the release time profile of a controlled-release form may be modified e.g. as follows: extended release, repeat action release, prolonged release and sustained release.

For the purpose of specification, "controlled release" preferably means a product in which the release of pharmacologically active ingredient over time is controlled by the type and composition of the formulation. For the purpose of specification "extended release" preferably means a product in which the release of pharmacologically active ingredient is delayed for a finite lag time, after which release is unhindered. For the purpose of specification "repeat action release" preferably means a product in which a first portion of pharmacologically active ingredient is released initially, followed by at least one further portion of pharmacologically active ingredient being released subsequently. For the purpose of specification "prolonged release" preferably means a product in which the rate of release of pharmacologically active ingredient from the formulation after administration has been reduced over time, in order to maintain therapeutic activity, to reduce toxic effects, or for some other therapeutic purpose. For the purpose of specification "sustained release" preferably means a way of formulating a medicine so that it is released into the body steadily, over a long period of time, thus reducing the dosing frequency. For further details, reference may be made, for example, to K.H. Bauer, Lehrbuch der Pharmazeutischen Technologie, 6th edition, WVG Stuttgart, 1999; and Eur. Ph.

Preferably, under physiological conditions the pharmaceutical dosage form according to the invention has released after 30 minutes 0.1 to 75%, after 240 minutes 0.5 to 95%, after 480 minutes 1.0 to 100% and after 720 minutes 2.5 to 100% of the pharmacologically active ingredient. Further preferred release profiles R₁ to R₆ are summarized in the table here below [all data in wt.-% of released pharmacologically active ingredient]:

| time | R₁ | R₂ | R₃ | R₄ | R₅ | R₆ |
|---|---|---|---|---|---|---|
| 60 min | 0-30 | 0-50 | 0-50 | 15-25 | 20-30 | 20-50 |
| 120 min | 0-40 | 0-75 | 0-75 | 25-40 | 35-50 | 40-75 |
| 240 min | 3-55 | 3-95 | 10-95 | 40-70 | 55-75 | 60-95 |
| 480 min | 10-65 | 10-100 | 35-100 | 60-90 | 80-95 | 80-100 |
| 720 min | 20-75 | 20-100 | 55-100 | 70-100 | 90-100 | 90-100 |
| 960 min | 30-88 | 30-100 | 70-100 | >80 | 95-100 | |
| 1,440 min | 50-100 | 50-100 | >90 | | | |
| 2,160 min | >80 | >80 | | | | |

Further preferred release profiles R₁ to R₆ are summarized in the table here below [all data in wt.-% of released pharmacologically active ingredient]:

| time | R₇ | R₈ | R₉ | R₁₀ | R₁₁ | R₁₂ |
|---|---|---|---|---|---|---|
| 30 min | 17.5±7.5 | 17.5±6.5 | 17.5±5.5 | 17.5±4.5 | 17.5±3.5 | 17.5±2.5 |
| 60 min | 27.0±8.0 | 27.0±7.0 | 27.0±6.0 | 27.0±5.0 | 27.0±4.0 | 27.0±3.0 |
| 120 min | 41.5±9.5 | 41.5±8.5 | 41.5±7.5 | 41.5±6.5 | 41.5±5.5 | 41.5±4.5 |
| 240 min | 64.5±12.5 | 64.5±11.5 | 64.5±10.5 | 64.5±9.5 | 64.5±8.5 | 64.5±7.5 |
| 480 min | 88.0±12.0 | 88.0±11.0 | 88.0±10.0 | 88.0±9.0 | 88.0±8.0 | 88.0±7.0 |
| 720 min | 96.0±9.0 | 96.0±8.0 | 96.0±7.0 | 96.0±6.0 | 96.0±5.0 | 96.0±4.0 |
| 840 min | 97.5±7.5 | 97.5±6.5 | 97.5±5.5 | 97.5±4.5 | 97.5±3.5 | 97.5±2.5 |

Preferably, the release profile of the pharmaceutical dosage form according to the invention is stable upon storage, preferably upon storage at elevated temperature, e.g. 40°C, for 3 months in sealed containers. In this regard "stable" means that when comparing the initial release profile with the release profile after storage, at any given time point the release profiles deviate from one another by not more than 20%, more preferably not more than 15%, still more preferably not more than 10%, yet more preferably not more than 7.5%, most preferably not more than 5.0% and in particular not more than 2.5%.

Preferably, under *in vitro* conditions the pharmaceutical dosage form has released after 0.5 h 1.0 to 35 wt.-%, after 1 h 5.0 to 45 wt.-%, after 2 h 10 to 60 wt.-%, after 4 h at least 15 wt.-%, after 6 h at least 20 wt.-%, after 8 h at least 25 wt.-% and after 12 h at least 30 wt.-% of the pharmacologically active ingredient that was originally contained in the pharmaceutical dosage form.

Suitable *in vitro* conditions are known to the skilled artisan. In this regard it can be referred to, e.g., the Eur. Ph. Preferably, the release profile is measured under the following conditions: Paddle apparatus equipped with sinker, 75 rpm, 37±5 °C, 600 mL simulated intestinal fluid pH 6.8 (phosphate buffer) or pH 4.5. In a preferred embodiment, the rotational speed of the paddle is increased to 100 rpm.

In a preferred embodiment, the pharmaceutical dosage form according to the invention is adapted for administration once daily. In another preferred embodiment, the pharmaceutical dosage form according to the invention is adapted for administration twice daily. In still another preferred embodiment, the pharmaceutical dosage form according to the invention is adapted for administration thrice daily.

For the purpose of specification, "twice daily" means equal or nearly equal time intervals, i.e., about every 12 hours, or different time intervals, e.g., 8 and 16 hours or 10 and 14 hours, between the individual administrations.

For the purpose of specification, "thrice daily" means equal or nearly equal time intervals, i.e., about every 8 hours, or different time intervals, e.g., 6, 6 and 12 hours; or 7, 7 and 10 hours, between the individual administrations.

A skilled person is fully aware that the above administration regimens twice daily or thrice daily, respectively, require an adaption of the dose of the pharmacologically active ingredient contained in the pharmaceutical dosage forms so that the total administered daily dose of the pharmacologically active ingredient, i.e. the sum of the doses contained in two or three, respectively, adapted pharmaceutical dosage forms, does not exceed the dose of the pharmacologically active ingredient contained in the dosage form which is adapted for administration once daily.

Preferably, the pharmaceutical dosage form according to the invention releases after 5 h at most 99%, more preferably at most 90%, still more preferably at most 75%, and most preferably at most 60% of the pharmacologically active ingredient.

In a particular preferred embodiment,
- the thermoformed pharmaceutical dosage form is prepared by melt-extrusion; and/or
- the pharmaceutical dosage form exhibits a breaking strength of at least 300 N; and/or
- the pharmaceutical dosage form is adapted for administration once daily, twice daily or thrice daily; and/or
- the pharmacologically active ingredient is selected from the group of opioids and opioid derivatives; and/or
- the polyalkylene oxide is selected from polymethylene oxide, polyethylene oxide and polypropylene oxide, or copolymers or mixtures thereof; having a weight average molecular weight (M_{W}) of more than 200,000 g/mol, preferably of at least 500,000 g/mol, more preferably within the range of from 1,000,000 g/mol to 10,000,000 g/mol; and/or
- the pharmaceutical dosage form contains a polyalkylene oxide composition including the polyalkylene oxide and the zinc component; and/or
- the content of said zinc component is at least 1 ppm and at most 10,000 ppm relative to the total weight of the pharmaceutical dosage form; and/or
- the content of the zinc component in the pharmaceutical dosage form is in the range of from 0.01 to 1 mol-% based on the zinc atom content per mol of the alkylene oxide units contained in the polyalkylene oxide; and/or
- the polyalkylene oxide is obtainable by polymerizing alkylene oxide in presence of the zinc component; and/or
- the polyalkylene oxide is obtainable by polymerizing alkylene oxide in presence of the zinc component, wherein the amount of said zinc component is in the range of from 0.01 to 1 mol-% based on the zinc atom content per mol of alkylene oxide; and/or
Ideally, the pharmaceutical dosage form according to the invention contains no substances which irritate the nasal passages and/or pharynx, i.e. substances which, when administered via the nasal passages and/or pharynx, bring about a physical reaction which is either so unpleasant for the patient that he/she does not wish to or cannot continue administration, for example burning, or physiologically counteracts taking of the corresponding pharmacologically active ingredient, for example due to increased nasal secretion or sneezing. Further examples of substances which irritate the nasal passages and/or pharynx are those which cause burning, itching, urge to sneeze, increased formation of secretions or a combination of at least two of these stimuli. Corresponding substances and the quantities thereof which are conventionally to be used are known to the person skilled in the art. Some of the substances which irritate the nasal passages and/or pharynx are accordingly based on one or more constituents or one or more plant parts of a hot substance pharmacologically active ingredient. Corresponding hot substance pharmacologically active ingredients are known per se to the person skilled in the art and are described, for example, in "Pharmazeutische Biologie - Drogen und ihre Inhaltsstoffe" by Prof. Dr. Hildebert Wagner, 2nd., revised edition, Gustav Fischer Verlag, Stuttgart-New York, 1982, pages 82 et seq.. The corresponding description is hereby introduced as a reference. The pharmaceutical dosage form furthermore preferably contains no antagonists for the pharmacologically active ingredient, preferably no antagonists against psychotropic substances, in particular no antagonists against opioids. Antagonists suitable for a given pharmacologically active ingredient are known to the person skilled in the art and may be present as such or in the form of corresponding derivatives, in particular esters or ethers, or in each case in the form of corresponding physiologically acceptable compounds, in particular in the form of the salts or solvates thereof. The pharmaceutical dosage form according to the invention preferably contains no antagonists selected from among the group comprising naloxone, naltrexone, nalmefene, nalide, nalmexone, nalorphine or naluphine, in each case optionally in the form of a corresponding physiologically acceptable compound, in particular in the form of a base, a salt or solvate; and no neuroleptics, for example a compound selected from among the group comprising haloperidol, promethacine, fluphenazine, perphenazine, levomepromazine, thioridazine, perazine, chlorpromazine, chlorprothixine, zuclopenthixol, flupentixol, prothipendyl, zotepine, benperidol, pipamperone, melperone and bromperidol.

The pharmaceutical dosage form furthermore preferably contains no emetic. Emetics are known to the person skilled in the art and may be present as such or in the form of corresponding derivatives, in particular esters or ethers, or in each case in the form of corresponding physiologically acceptable compounds, in particular in the form of the salts or solvates thereof. The pharmaceutical dosage form according to the invention preferably contains no emetic based on one or more constituents of ipecacuanha (ipecac) root, for example based on the constituent emetine, as are, for example, described in "Pharmazeutische Biologie - Drogen und ihre Inhaltsstoffe" by Prof. Dr. Hildebert Wagner, 2nd, revised edition, Gustav Fischer Verlag, Stuttgart, New York, 1982. The corresponding literature description is hereby introduced as a reference. The pharmaceutical dosage form preferably also contains no apomorphine as an emetic. Preferably, the pharmaceutical dosage form according to the invention does not contain a zinc component as an emetic. Particularly preferably, the pharmaceutical dosage form according to the invention does not contain zinc sulfate, which is useful as an emetic.

Finally, the pharmaceutical dosage form preferably also contains no bitter substance. Bitter substances and the quantities effective for use may be found in US-2003/0064099 A1, the corresponding disclosure which is hereby introduced as a reference. Examples of bitter substances are aromatic oils, such as peppermint oil, eucalyptus oil, bitter almond oil, menthol, fruit aroma substances, aroma substances from lemons, oranges, limes, grapefruit or mixtures thereof, and/or denatonium benzoate.

The pharmaceutical dosage form according to the invention is tamper resistant. Preferably, tamper-resistance is achieved based on the mechanical properties of the pharmaceutical dosage form so that comminution is avoided or at least substantially impeded. The pharmaceutical dosage form according to the invention preferably has a breaking strength of at least 300 N. According to the invention, the term comminution means the pulverization of the pharmaceutical dosage form using conventional means usually available to an abuser, for example a pestle and mortar, a hammer, a mallet or other conventional means for pulverizing under the action of force. Thus, tamper-resistance preferably means that pulverization of the pharmaceutical dosage form using conventional means is avoided or at least substantially impeded.

Preferably, the mechanical properties of the pharmaceutical dosage form according to the invention, particularly its breaking strength, substantially rely on the presence and spatial distribution of the polyalkylene oxide and of the polyalkylene oxide composition, respectively, although their mere presence does typically not suffice in order to achieve said properties. The advantageous mechanical properties of the pharmaceutical dosage form according to the invention may not automatically be achieved by simply processing pharmacologically active ingredient, polyalkylene oxide, a zinc component, and optionally further excipients by means of conventional methods for the preparation of pharmaceutical dosage forms. In fact, usually suitable apparatuses must be selected for the preparation and critical processing parameters must be adjusted, particularly pressure/force, temperature and time. Thus, even if conventional apparatuses are used, the process protocols usually must be adapted in order to meet the required criteria.

In general, the pharmaceutical dosage forms exhibiting the desired properties may be obtained only if, during preparation of the pharmaceutical dosage form,
- suitable components
- in suitable amounts
   are exposed to
- sufficient pressure
- at sufficient temperature
- for a sufficient period of time.

Thus, regardless of the apparatus used, the process protocols must be adapted in order to meet the required criteria. Therefore, the breaking strength is separable from the composition.

The pharmaceutical dosage form according to the invention has a breaking strength of at least 300 N, preferably at least 400 N, more preferably at least 500 N, still more preferably at least 600 N, yet more preferably at least 700 N, even more preferably at least 800 N most preferably at least 900 N and in particular at least 1,000 N. The pharmaceutical dosage form according to the invention may have a breaking strength of at least 1,100 N, preferably at least 1,200 N, more preferably at least 1,300 N, most preferably at least 1,400 N and in particular at least 1,500 N.

The "breaking strength" (resistance to crushing) of a pharmaceutical dosage form is known to the skilled person. In this regard it can be referred to, e.g., W.A. Ritschel, Die Tablette, 2. Auflage, Editio Cantor Verlag Aulendorf, 2002; H Liebermann et al., Pharmaceutical dosage forms: Tablets, Vol. 2, Informa Healthcare; 2 edition, 1990; and Encyclopedia of Pharmaceutical Technology, Informa Healthcare; 1 edition.

For the purpose of specification, the breaking strength is preferably defined as the amount of force that is necessary in order to fracture the pharmaceutical dosage form (= breaking force). Therefore, for the purpose of specification the pharmaceutical dosage form does preferably not exhibit the desired breaking strength when it breaks, i.e., is fractured into at least two independent parts that are separated from one another. In another preferred embodiment, however, the pharmaceutical dosage form is regarded as being broken if the force decreases by 25% (threshold value) of the highest force measured during the measurement (see below).

The pharmaceutical dosage forms according to the invention are distinguished from conventional pharmaceutical dosage forms in that, due to their breaking strength, they cannot be pulverized by the application of force with conventional means, such as for example a pestle and mortar, a hammer, a mallet or other usual means for pulverization, in particular devices developed for this purpose (tablet crushers). In this regard "pulverization" means crumbling into small particles that would immediately release the pharmacologically active ingredient in a suitable medium. Avoidance of pulverization virtually rules out oral or parenteral, in particular intravenous or nasal abuse.

Conventional tablets typically have a breaking strength well below 200 N in any direction of extension. The breaking strength of conventional round tablets may be estimated according to the following empirical formula: *Breaking Strength* [in N] = *10 x Diameter Of The Tablet* [in mm]. Thus, according to said empirical formula, a round tablet having a breaking strength of at least 300 N would require a diameter of at least 30 mm). Such a tablet, however, could not be swallowed. The above empirical formula preferably does not apply to the pharmaceutical dosage forms of the invention, which are not conventional but rather special.

Further, the actual mean chewing force is about 220 N (cf., e.g., P.A. Proeschel et al., J Dent Res, 2002, 81(7), 464-468). This means that conventional tablets having a breaking strength well below 200 N may be crushed upon spontaneous chewing, whereas the pharmaceutical dosage forms according to the invention may not.

Still further, when applying a gravitational acceleration of about 9.81 m/s², 300 N correspond to a gravitational force of more than 30 kg, i.e. the pharmaceutical dosage forms according to the invention can preferably withstand a weight of more than 30 kg without being pulverized.

Methods for measuring the breaking strength of a pharmaceutical dosage form are known to the skilled artisan. Suitable devices are commercially available.

For example, the breaking strength (resistance to crushing) can be measured in accordance with the Eur. Ph. 5.0, 2.9.8 or 6.0, 2.09.08 "Resistance to Crushing of Tablets". The test is intended to determine, under defined conditions, the resistance to crushing of tablets, measured by the force needed to disrupt them by crushing. The apparatus consists of 2 jaws facing each other, one of which moves towards the other. The flat surfaces of the jaws are perpendicular to the direction of movement. The crushing surfaces of the jaws are flat and larger than the zone of contact with the tablet. The apparatus is calibrated using a system with a precision of 1 Newton. The tablet is placed between the jaws, taking into account, where applicable, the shape, the break-mark and the inscription; for each measurement the tablet is oriented in the same way with respect to the direction of application of the force (and the direction of extension in which the breaking strength is to be measured). The measurement is carried out on 10 tablets, taking care that all fragments of tablets have been removed before each determination. The result is expressed as the mean, minimum and maximum values of the forces measured, all expressed in Newton.

A similar description of the breaking strength (breaking force) can be found in the USP. The breaking strength can alternatively be measured in accordance with the method described therein where it is stated that the breaking strength is the force required to cause a tablet to fail (i.e., break) in a specific plane. The tablets are generally placed between two platens, one of which moves to apply sufficient force to the tablet to cause fracture. For conventional, round (circular cross-section) tablets, loading occurs across their diameter (sometimes referred to as diametral loading), and fracture occurs in the plane. The breaking force of tablets is commonly called hardness in the pharmaceutical literature; however, the use of this term is misleading. In material science, the term hardness refers to the resistance of a surface to penetration or indentation by a small probe. The term crushing strength is also frequently used to describe the resistance of tablets to the application of a compressive load. Although this term describes the true nature of the test more accurately than does hardness, it implies that tablets are actually crushed during the test, which is often not the case.

Alternatively, the breaking strength (resistance to crushing) can be measured in accordance with WO 2005/ 016313, WO 2005/016314, and WO 2006/082099, which can be regarded as a modification of the method described in the Eur. Ph. The apparatus used for the measurement is preferably a "Zwick Z 2.5" materials tester, Fₘₐₓ = 2.5 kN with a maximum draw of 1,150 mm, which should be set up with one column and one spindle, a clearance behind of 100 mm and a test speed adjustable between 0.1 and 800 mm/min together with testControl software. Measurement is performed using a pressure piston with screw-in inserts and a cylinder (diameter 10 mm), a force transducer, Fₘₐₓ. 1 kN, diameter = 8 mm, class 0.5 from 10 N, class 1 from 2 N to ISO 7500-1, with manufacturer's test certificate M according to DIN 55350-18 (Zwick gross force Fₘₐₓ = 1.45 kN) (all apparatus from Zwick GmbH & Co. KG, Ulm, Germany) with Order No BTC-FR 2.5 TH. D09 for the tester, Order No BTC-LC 0050N. P01 for the force transducer, Order No BO 70000 S06 for the centering device.

In a preferred aspect, the breaking strength is measured by means of a breaking strength tester e.g. Sotax^{®}, type HT100 or type HT1 (Allschwil, Switzerland). Both, the Sotax^{®} HT100 and the Sotax^{®} HT1 can measure the breaking strength according to two different measurement principles: constant speed (where the test jaw is moved at a constant speed adjustable from 5-200 mm/min) or constant force (where the test jaw increases force linearly adjustable from 5-100 N/sec). In principle, both measurement principles are suitable for measuring the breaking strength of the pharmaceutical dosage form according to the invention. Preferably, the breaking strength is measured at constant speed, preferably at a constant speed of 120 mm/min. The pharmaceutical dosage form is regarded as being broken if it is fractured into at least two separate pieces.

The pharmaceutical dosage form according to the invention preferably exhibits mechanical strength over a wide temperature range, in addition to the breaking strength (resistance to crushing) optionally also sufficient hardness, impact resistance, impact elasticity, tensile strength and/or modulus of elasticity, optionally also at low temperatures (e.g. below -24 °C, below -40 °C or in liquid nitrogen), for it to be virtually impossible to pulverize by spontaneous chewing, grinding in a mortar, pounding, etc. Thus, preferably, the comparatively high breaking strength of the pharmaceutical dosage form according to the invention is maintained even at low or very low temperatures, e.g., when the pharmaceutical dosage form is initially chilled to increase its brittleness, for example to temperatures below -25°C, below -40 °C or even in liquid nitrogen.

The pharmaceutical dosage form according to the invention is characterized by a certain degree of breaking strength. This does not mean that the pharmaceutical dosage form must also exhibit a certain degree of hardness. Hardness and breaking strength are different physical properties. Therefore, the tamper resistance of the pharmaceutical dosage form does not necessarily depend on the hardness of the pharmaceutical dosage form. For instance, due to its breaking strength, impact strength, elasticity modulus and tensile strength, respectively, the pharmaceutical dosage form can preferably be deformed, e.g. plastically, when exerting an external force, for example using a hammer, but cannot be pulverized, i.e., crumbled into a high number of fragments. In other words, the pharmaceutical dosage form according to the invention is characterized by a certain degree of breaking strength, but not necessarily also by a certain degree of form stability.

Therefore, in the meaning of the specification, a pharmaceutical dosage form that is deformed when being exposed to a force in a particular direction of extension but that does not break (plastic deformation or plastic flow) is preferably to be regarded as having the desired breaking strength in said direction of extension. The disclosure relates to a tamper-resistant pharmaceutical dosage form having a retarded release profile, especially a tamper-resistant oral dosage form having a retarded release profile, particularly a tamper-resistant tablet having a retarded release profile comprising at least one pharmaceutically pharmacologically active ingredient (pharmacologically active compound) with potential for abuse.

The pharmaceutical dosage form according to the invention may be produced by different processes, the particularly preferred of which are explained in greater detail below. Several suitable processes have already been described in the prior art. In this regard it can be referred to, e.g., WO 2005/ 016313, WO 2005/016314, WO 2005/063214, WO 2005/102286, WO 2006/002883, WO 2006/002884, WO 2006/002886, WO 2006/082097, and WO 2006/082099.

The present invention also relates to pharmaceutical dosage forms that are obtainable by any of the processes described here below.

In a preferred embodiment, the pharmaceutical dosage form is manufactured in a process comprising the steps of
(a) mixing a pharmacologically active ingredient, a polyalkylene oxide composition comprising a polyalkylene oxide having a weight average molecular weight of more than 200,000 g/mol and a zinc component, and optionally present excipients, wherein the content of the zinc component is at least 1 ppm, relative to the total weight of the mixture prepared in step (a);
(b) press-forming the mixture obtained in step (a) with preceding, simultaneous, or subsequent exposure to heat.

In general, the process for the production of the pharmaceutical dosage form according to the invention preferably comprises the following steps:
(a) mixing all ingredients;
(b) optionally pre-forming the mixture obtained from step (a), preferably by applying heat and/or force to the mixture obtained from step (a), the quantity of heat supplied preferably not being sufficient to heat the polyalkylene oxide up to its softening point;
(c) hardening the mixture by applying heat and force, it being possible to supply the heat during and/or before the application of force and the quantity of heat supplied being sufficient to heat the polyalkylene oxide at least up to its softening point;
(d) optionally singulating the hardened mixture;
(e) optionally shaping the pharmaceutical dosage form; and
(f) optionally providing a film coating.

Heat may be supplied directly, e.g. by contact or by means of hot gas such as hot air, or with the assistance of ultrasound. Force may be applied and/or the pharmaceutical dosage form may be shaped for example by direct tabletting or with the assistance of a suitable extruder, particularly by means of a screw extruder equipped with two screws (twin-screw-extruder) or by means of a planetary gear extruder.

Preferably, hot-melt extrusion is performed in the absence of additional water.

The final shape of the pharmaceutical dosage form may either be provided during the hardening of the mixture by applying heat and force (step (c)) or in a subsequent step (step (e)). In both cases, the mixture of all components is preferably in the plastified state, i.e. preferably, shaping is performed at a temperature at least above the softening point of the polyalkylene oxide. However, extrusion at lower temperatures, e.g. ambient temperature, is also possible and may be preferred.

Shaping can be performed, e.g., by means of a tabletting press comprising die and punches of appropriate shape.

A particularly preferred process for the manufacture of the pharmaceutical dosage form of the invention involves hot-melt extrusion. In this process, the pharmaceutical dosage form according to the invention is produced by thermoforming with the assistance of an extruder, preferably without there being any observable consequent discoloration of the extrudate.

This process is characterized in that
a) all components are mixed,
b) the resultant mixture is heated in the extruder at least up to the softening point of the polyalkylene oxide and extruded through the outlet orifice of the extruder by application of force,
c) the still plastic extrudate is singulated and formed into the pharmaceutical dosage form or
d) the cooled and optionally reheated singulated extrudate is formed into the pharmaceutical dosage form.

Mixing of the components according to process step a) may also proceed in the extruder.

The components may also be mixed in a mixer known to the person skilled in the art. The mixer may, for example, be a roll mixer, shaking mixer, shear mixer or compulsory mixer.

The preferably molten mixture which has been heated in the extruder at least up to the softening point of the polyalkylene oxide is extruded from the extruder through a die with at least one bore.

The extrusion process according to the invention requires the use of suitable extruders, preferably screw extruders. Screw extruders which are equipped with two screws (twin-screw-extruders) are particularly preferred.

The extrusion is preferably performed so that the expansion of the strand due to extrusion is not more than 30%, i.e. that when using a die with a bore having a diameter of e.g. 6 mm, the extruded strand should have a diameter of not more than 8 mm. More preferably, the expansion of the strand is not more than 25%, still more preferably not more than 20%, most preferably not more than 15% and in particular not more than 10%.

Preferably, extrusion is performed in the absence of water, i.e., no water is added. However, traces of water (e.g., caused by atmospheric humidity) may be present.

The extruder preferably comprises at least two temperature zones, with heating of the mixture at least up to the softening point of the polyalkylene oxide proceeding in the first zone, which is downstream from a feed zone and optionally mixing zone. The throughput of the mixture is preferably from 1.0 kg to 30 kg/hour, more preferably 1.0 kg to 15 kg/hour. In one aspect, the throughput is from 1 to 3.5 kg/hour. In another aspect, the throughput is from 4 to 15 kg/hour. Generally, the die head pressure is within the range of from 25 to 200 bar, more preferably 25 to 100 bar. The die head pressure can be adjusted *inter alia* by die geometry, temperature profile and extrusion speed.

The die geometry or the geometry of the bores is freely selectable. The die or the bores may accordingly exhibit a round, oblong or oval cross-section, wherein the round cross-section preferably has a diameter of 0.1 mm to 15 mm and the oblong cross-section preferably has a maximum lengthwise extension of 21 mm and a crosswise extension of 10 mm. Preferably, the die or the bores have a round cross-section. The casing of the extruder used according to the invention may be heated or cooled. The corresponding temperature control, i.e. heating or cooling, is so arranged that the mixture to be extruded exhibits at least an average temperature (product temperature) corresponding to the softening temperature of the polyalkylene oxide and does not rise above a temperature at which the pharmacologically active ingredient to be processed may be damaged. Preferably, the temperature of the mixture to be extruded is adjusted to below 180 °C, preferably below 150 °C, but at least to the softening temperature of the polyalkylene oxide. Typical extrusion temperatures are 120 °C and 130 °C. The extruder torque may be within the range of from 30 to 95%. Extruder torque can be adjusted inter alia by die geometry, temperature profile and extrusion speed.

After extrusion of the molten mixture and optional cooling of the extruded strand or extruded strands, the extrudates are preferably singulated. This singulation may preferably be performed by cutting up the extrudates by means of revolving or rotating knives, water jet cutters, wires, blades or with the assistance of laser cutters.

Preferably, intermediate or final storage of the optionally singulated extrudate or the final shape of the pharmaceutical dosage form according to the invention is performed under oxygen-free atmosphere which may be achieved, e.g., by means of oxygen-scavengers.

The singulated extrudate may be press-formed into tablets in order to impart the final shape to the pharmaceutical dosage form.

The application of force in the extruder onto the at least plasticized mixture is adjusted by controlling the rotational speed of the conveying device in the extruder and the geometry thereof and by dimensioning the outlet orifice in such a manner that the pressure necessary for extruding the plasticized mixture is built up in the extruder, preferably immediately prior to extrusion. The extrusion parameters which, for each particular composition, are necessary to give rise to a pharmaceutical dosage form with desired mechanical properties, may be established by simple preliminary testing.

For example but not limiting, extrusion may be performed by means of a twin-screw-extruder type ZSE 18 or ZSE27 (Leistritz, Nürnberg, Germany), screw diameters of 18 or 27 mm. Screws having eccentric ends may be used. A heatable die with a round bore having a diameter of 7, 8, or 9 mm may be used. The extrusion parameters may be adjusted e.g. to the following values: rotational speed of the screws: 120 Upm; delivery rate 2 kg/h for a ZSE 18 or 8 kg/h for a ZSE27; product temperature: in front of die 125 °C and behind die 135 °C; and jacket temperature: 110 °C. In another preferred embodiment, extrusion is performed by means of a twin-screw-extruder, type ZSE 27 PH 40 D (Leistritz, Nürnberg, Germany), with a diameter of the extrusion die of 5.5 mm or 7 mm at a temperature of the extrusion die of 135°C).

Preferably, extrusion is performed by means of twin-screw-extruders or planetary-gear-extruders, twin-screw extruders (co-rotating or contra-rotating) being particularly preferred.

The pharmaceutical dosage form may be produced by thermoforming with the assistance of an extruder without any observable consequent discoloration of the extrudates.

The process for the preparation of the pharmaceutical dosage form is preferably performed continuously. Preferably, the process involves the extrusion of a homogeneous mixture of all components. It is particularly advantageous if the thus obtained intermediate, e.g. the strand obtained by extrusion, exhibits uniform properties. Particularly desirable are uniform density, uniform distribution of the pharmacologically active ingredient, uniform mechanical properties, uniform porosity, uniform appearance of the surface, etc. Only under these circumstances the uniformity of the pharmacological properties, such as the stability of the release profile, may be ensured and the amount of rejects can be kept low.

A further aspect of the disclosure relates to the use of a pharmacologically active ingredient for the manufacture of the pharmaceutical dosage form as described above for the treatment of pain.

A further aspect of the disclosure relates to the use of a pharmaceutical dosage form as described above for avoiding or hindering the abuse of the pharmacologically active ingredient contained therein.

A further aspect of the disclosure relates to the use of a pharmaceutical dosage form as described above for avoiding or hindering the unintentional overdose of the pharmacologically active ingredient contained therein.

In this regard, the invention also discloses the use of a pharmacologically active ingredient as described above and a polyalkylene oxide composition as described above for the manufacture of the pharmaceutical dosage form according to the invention for the prophylaxis and/or the treatment of a disorder, thereby preventing an overdose of the pharmacologically active ingredient, particularly due to comminution of the pharmaceutical dosage form by mechanical action.

Further, the disclosure relates to a method for the prophylaxis and/or the treatment of a disorder comprising the administration of the pharmaceutical dosage form according to the invention, while at the same time preventing an overdose of the pharmacologically active ingredient, particularly due to comminution of the pharmaceutical dosage form by mechanical action. Preferably, the mechanical action is selected from the group consisting of chewing, grinding in a mortar, pounding, and using apparatuses for pulverizing conventional pharmaceutical dosage forms.

The following examples further illustrate the invention but are not to be construed as limiting its scope:

### Example 1:

Four samples containing different types of polyethylene oxides were prepared by dispersing the polyethylene oxide (1.0 g) in water (99.0 g) using an IKA Ultra-Turrax T-25 disperser. Afterwards, the dispersion was stirred for 1 h using an IKA RCT basic safety control magnetic stirrer. When using PEO 20NF, PEO 18NF and PEO Coagulant, dispersion was repeated followed by stirring for 15 min. After four days, gel formation was complete and the pH values were measured using a Knick type 765 laboratory pH meter. All measurements were conducted at 25°C. The pH values of the different samples are summarized in the following table:

| polyethylene oxide product | | M_{W} [g·mol⁻¹] | viscosity [mPa·s] | pH |
|---|---|---|---|---|
| inventive | PEO 18NF | 4,500,000 | 5,500-7,500 | 6.31 |
| | PEO 20NF | 5,000,000 | 7,500-10,000 | 6.41 |
| comparative | Polyox^{®} Coagulant | 5,000,000 | 5,500-6,280 | 7.78 |
| | Polyox^{®} WSR303 | 7,000,000 | 7,500-10,000 | 8.25 |

The aqueous dispersions of polyethylene oxides PEO 20NF and PEO 18NF (commercialized by Sumitomo) had pH values of 6.41 and 6.31, respectively. According to the product specification, zinc was present at a zinc content below 1,000 ppm.

The pH values of the comparative examples Polyox^{®} WSR303 and Polyox^{®} Coagulant (commercialized by Dow) amounted to 8.25 and 7.78, respectively. According to the manufacturer, zinc is not used in any part of the Polyox^{®} WSR301 manufacturing process and although not specifically tested, it is not expected to be present. Polyox^{®} WSR301 is a homologue of Polyox^{®} WSR303.

### Example 2:

A stability study was conducted comparing pharmaceutical dosage forms manufactured at commercial scale (batch sizes 15 kg or 85 kg) using polyethylene oxide containing zinc (inventive examples) or zinc-free polyethylene oxide (comparative examples). All tablets had a high breaking strength of above 500 N.

Two different formulations were prepared, containing 56.29 wt.-% and 35.00 wt.-% of polyethylene oxide (zinc containing or zinc free), respectively.

The zinc-containing polyethylene oxide was PEO 20NF (7,500-10,000 mPa·s) of Sumitomo and is referred to in the following as "PEO, Zn containing". The zinc-free polyethylene oxide was Polyox^{®} WSR303 (7,500-10,000 mPa·s) of Dow and is referred to in the following as "PEO, Zn free".

In order to exclude batch-specific differences, each formulation was prepared at least twice by using the same type of chemical compounds in the same amounts only partially differing in the commercially available batches, i.e. lot numbers.

According to the manufacturer's certificates of analysis, the Zn content in the zinc-containing polyethylene oxide batches was about 0.086%.

The mixtures of the pharmacologically active ingredient, polyalkylene oxide and excipients were extruded by means of a twin-screw-extruder (type ZSE 27 PH 40 D, diameter of extrusion die 5.5 mm for the compositions I/C 1-x and 7 mm for the compositions I/C 2-x, temperature of extrusion die 135°C).

Details on the formulations tested can be found in the following table:

| **1:** amount in % up to 103% with tablet core equaling 100% (amount per tablet in mg with total weight of tablet of 412.00 mg) | | | | | | | | |
|---|---|---|---|---|---|---|---|---|
| | PEO **Zn containing** | PEO **Zn free** | Tapentadol-HCl | Hypromellose 100000 mPas, Ph.Eur. | Macrogol 6000, Ph.Eur. | α-Tocopherol | PEG | Opadry® II white 85F18422 |
| **I 1-1** and **I 1-2** | 56.29 (225.16) | - | 14.56 (58.24) | 14.00 (56.00) | 14.08 (56.31) | 0.15 (0.60) | 0.92 (3.69) | 3.00 (12.00) |
| **C 1-1, C 1-2, C 1-3** and **C 1-4** | - | 56.29 (225.16) | | | | | | |

| **2:** amount in % up to 103% with tablet core equaling 100% (amount per tablet in mg with total weight of tablet of 721.00 mg) | | | | | | | | |
|---|---|---|---|---|---|---|---|---|
| | PEO **Zn containing** | PEO **Zn free** | Tapentadol-HCl | Hypromellose 100000 mPas, Ph.Eur. | Macrogol 6000, Ph.Eur. | α-Tocopherol | PEG | Opadry® II red 85F35244 |
| **I 2-1** and **I 2-2** | 35.00 (245.00) | - | 41.60 (291.20) | 14.00 (98.00) | 8.69 (60.80) | 0.10 (0.70) | 0.61 (4.30) | 3.00 (21.00) |
| **C 2-1, C 2-2, C 2-3** and **C 2-4** | - | 35.00 (245.00) | | | | | | |

The film-coated tablets were packaged into blisters and put on stability. Storage conditions were 25°C/60% r.h., 30°C/75% r.h., and 40°C/75% r.h..

As indicative parameters for the degradation of polyethylene oxide viscosity and α-tocopherol consumption were identified. When the polymer chains detoriate, the viscosity of the aqueous gel of the tablet decreases. The degradation of polyethylene oxide is an oxidative process, therefore it is linked to a decrease in the content of the antioxidant α-tocopherol in the formulation.

In particular, the beneficial effects of the zinc containing dosage forms according to the invention become evident at the harsher storage conditions (40°C/75% r.h.).

After storage for 6 months at 40°C/75% r.h., the tablets still had a high breaking strength of above 500 N.

### Description of the method for determining the viscosity:

One or two tablets are cut into smaller pieces and accurately weighed (242 +/- 3 mg for I/C 1-x, 388 +/- 3 mg for I/C 2-x) and dissolved in 2 mL 2-Propanole and 10 mL water. Dissolving takes place over 72 hours while shaking on a mechanical agitator at 175 to 250 rpm. 2 mL of the formed solution are measured in the viscosimeter.

Apparatus: Thermo Scientific HAAKE RotoVisco1, equipped with a RV1 plate system and a C60/1° cone with a split of 0.052 mm. Temperature is 25.0°C +/- 0.1 °C. The measurement takes place in CR mode, starting at a shear rate of 0.0001/sec, ending at a shear rate of 200.01/sec with linear distribution. The duration is 180 sec, 180 data points are collected linear over the measurement. Measurement takes place at shear rates of 40.00, 80.00. 120.00, and 160.00 1/s. The result is reported as the viscosity in mPa·s at a shear rate of 160/s.

### Description of the method for the α-tocopherol assay:

The α-tocopherol content is determined by a HPLC method as follows:
Chromatographic System
Column: Lichrospher 100-5 RP8 250*4.0 mm 5µm or equivalent
Eluent: 10% Trifluor acetic acid 0.3% in water, 90 % Trifluor acetic acid 0.3% in acetonitrile
Detection: UV-210 nm
Flow: 1.5 mL/min
Injection volume: 50µL
Column temperature: 35°C

**Viscosity [mPa·s] @25°C/60% r.h.:**

| | content of PEO [%] | viscosity [mPa·s] | | | |
|---|---|---|---|---|---|
| | | start of storage | after 6 months | absolute change over 6 months | decrease over 6 months [%] |
| **I 1-1** | 56.29 (Zn containing) | 314 | 273 | -41 | 13 |
| **C 1-1** | 56.29 (Zn free) | 390 | 377 | -13 | 3 |
| **C 1-2** | | 375 | 305 | -70 | 19 |
| **C 1-4** | | 418 | 396 | -22 | 5 |
| **I 2-1** | 35.00 (Zn containing) | 402 | 361 | -41 | 10 |
| **I 2-2** | | 382 | 289 | -93 | 24 |
| **C 2-1** | 35.00 (Zn free) | 464 | 444 | -20 | 4 |
| **C 2-2** | | 443 | 405 | -38 | 9 |
| **C 2-3** | | 508 | 404 | -104 | 20 |
| **C 2-4** | | 506 | 400 | -106 | 21 |

**Viscosity [mPa·s] @ 30°C/75% r.h.:**

| | content of PEO [%] | viscosity [mPa·s] | | | | |
|---|---|---|---|---|---|---|
| | | start of storage | after 3 months | after 6 months | absolute change over 6 months | decrease over 6 months [%] |
| **I 1-1** | 56.29 (Zn containing) | 314 | 317 | 250 | -64 | 20 |
| **C 1-1** | 56.29 (Zn free) | 390 | 371 | 344 | -46 | 12 |
| **C 1-2** | | 375 | 324 | 301 | -74 | 20 |
| **C 1-3** | | 382 | 389 | 358 | -24 | 6 |
| **C 1-4** | | 418 | 367 | 333 | -85 | 20 |
| **I 2-2** | 35.00 (Zn containing) | 382 | 399 | 357 | -25 | 7 |
| **C 2-1** | 35.00 (Zn free) | 464 | 459 | 457 | -7 | 2 |
| **C 2-2** | | 443 | 440 | 378 | -65 | 15 |
| **C 2-3** | | 508 | 447 | 420 | -88 | 17 |
| **C 2-4** | | 506 | 444 | 395 | -111 | 22 |

**Viscosity [mPa·s] @ 40°C/75% r.h.:**

| | content of PEO [%] | viscosity [mPa·s] | | | | | |
|---|---|---|---|---|---|---|---|
| | | start of storage | after 1 month | after 3 months | after 6 months | absolute change over 6 months | decrease over 6 months [%] |
| **I 1-1** | 56.29 (Zn containing) | 314 | 344 | 327 | 308 | -6 | 2 |
| **I 1-2** | | 272 | 344 | 312 | 254 | -18 | 7 |
| **C 1-1** | 56.29 (Zn free) | 390 | 356 | 329 | 282 | -108 | 28 |
| **C 1-2** | | 375 | 415 | 304 | 263 | -112 | 30 |
| **C 1-3** | | 382 | 343 | 350 | 276 | -106 | 28 |
| **C 1-4** | | 418 | 400 | 400 | 270 | -148 | 35 |
| **I 2-1** | 35.00 (Zn containing) | 402 | 412 | 405 | 397 | -5 | 1 |
| **I 2-2** | | 382 | 396 | 387 | 359 | -23 | 6 |
| **C 2-1** | 35.00 (Zn free) | 464 | 477 | 430 | 356 | -108 | 23 |
| **C 2-2** | | 443 | 471 | 403 | 342 | -101 | 23 |
| **C 2-3** | | 508 | 481 | 411 | 372 | -136 | 27 |
| **C 2-4** | | 506 | 449 | 401 | 364 | -142 | 28 |

It becomes evident from the data summarized in above table, that the zinc containing dosage forms according to the invention display improved stability in comparison to dosage forms not containing zinc, in particular at accelerated storage conditions at 40°C and 75% r.h..

While all inventive examples showed a decrease in viscosity after 6 months of storage (40°C/75% r.h.) of only 1% (I 2-1) up to a maximum decrease of 7% (I 1-2), all comparative examples not containing zinc exhibited a decrease in viscosity of at least 23% (C 2-1 and C 2-2) up to 35% (C 1-4), relative to the respective viscosity at the start of the storage.

In the following, the content of α-tocopherol is given in wt.-% relative to the given theoretical content of α-tocopherol in the pharmaceutical dosage form.

For the purpose of specification, the "given theoretical content of α-tocopherol" shall refer to the weight content the α-tocopherol should have in theory according to the specification of the composition of the pharmaceutical dosage form.

**Content (wt.-%) α-tocopherol @25°C/60% r.h.**

| | content of PEO [%] | content of α-tocopherol | | | |
|---|---|---|---|---|---|
| | | start of storage [wt.-%] | after 6 months [wt.-%] | absolute change over 6 months [wt.-%] | decrease over 6 months [%] |
| **I 1-1** | 56.29 (Zn containing) | 94 | 90 | -4 | 4 |
| **I 1-2** | | 93 | 88 | -5 | 5 |
| **C 1-1** | 56.29 (Zn free) | 93 | 87 | -6 | 6 |
| **C 1-2** | | 97 | 91 | -6 | 6 |
| **C 1-3** | | 94 | 89 | -5 | 5 |
| **C 1-4** | | 97 | 90 | -7 | 7 |
| **I 2-1** | 35.00 (Zn containing) | 90 | 87 | -3 | 3 |
| **I 2-2** | | 89 | 87 | -2 | 2 |
| **C 2-1** | 35.00 (Zn free) | 87 | 85 | -2 | 2 |
| **C 2-2** | | 93 | 85 | -8 | 9 |
| **C 2-3** | | 89 | 87 | -2 | 2 |
| **C 2-4** | | 92 | 86 | -6 | 7 |

**Content (wt.-%) α-tocopherol @30°C/75% r.h.**

| | content of PEO [%] | content of α-tocopherol | | | | |
|---|---|---|---|---|---|---|
| | | start of storage [wt.-%] | after 3 months [wt.-%] | after 6 months [wt.-%] | absolute change over 6 months [wt.-%] | decrease over 6 months [%] |
| **I 1-1** | 56.29 (Zn containing) | 94 | 90 | 88 | -6 | 6 |
| **I 1-2** | | 93 | 89 | 86 | -7 | 8 |
| **C 1-1** | 56.29 (Zn free) | 93 | 89 | 83 | -10 | 11 |
| **C 1-2** | | 97 | 91 | 88 | -9 | 9 |
| **C 1-3** | | 94 | 92 | 86 | -8 | 9 |
| **C 1-4** | | 97 | 93 | 86 | -11 | 11 |
| **I 2-1** | 35.00 (Zn containing) | 90 | 88 | 85 | -5 | 6 |
| **I 2-2** | | 89 | 88 | 85 | -4 | 4 |
| **C 2-1** | 35.00 (Zn free) | 87 | 86 | 81 | -6 | 7 |
| **C 2-2** | | 93 | 87 | 80 | -13 | 14 |
| **C 2-3** | | 89 | 88 | 83 | -6 | 7 |
| **C 2-4** | | 92 | 88 | 82 | -10 | 11 |

**Content (wt.-%) α-tocopherol @40°C/75% r.h.**

| | content of PEO [%] | content of α-tocopherol | | | | | |
|---|---|---|---|---|---|---|---|
| | | start of storage [wt.-%] | after 1 month [wt.-%] | after 3 months [wt.-%] | after 6 months [wt.-%] | absolute change over 6 months [wt.-%] | decrease over 6 months [%] |
| **I 1-1** | 56.29 (Zn containing) | 94 | 93 | 86 | 79 | -15 | 16 |
| **I 1-2** | | 93 | 93 | 84 | 77 | -16 | 17 |
| **C 1-1** | 56.29 (Zn free) | 93 | 89 | 85 | 73 | -20 | 22 |
| **C 1-2** | | 97 | 93 | 88 | 78 | -19 | 20 |
| **C 1-3** | | 94 | 94 | 89 | 72 | -22 | 23 |
| **C 1-4** | | 97 | 93 | 90 | 76 | -21 | 22 |
| **I 2-1** | 35.00 (Zn containing) | 90 | 89 | 85 | 76 | -14 | 16 |
| **I 2-2** | | 89 | 91 | 84 | 75 | -14 | 16 |
| **C 2-1** | 35.00 (Zn free) | 87 | 90 | 85 | 66 | -21 | 24 |
| **C 2-2** | | 93 | 91 | 79 | 64 | -29 | 31 |
| **C 2-3** | | 89 | 90 | 83 | 71 | -18 | 20 |
| **C 2-4** | | 92 | 89 | 82 | 69 | -23 | 25 |

In the data presented it is shown that for the inventive formulations the degradation of the polyethylene oxide is reduced compared to the comparative formulations.

It becomes evident from the data summarized in above table, that the zinc containing dosage forms according to the invention display improved stability in comparison to dosage forms not containing zinc, in particular at accelerated storage conditions at 40°C and 75% r.h..

While all inventive examples showed a decrease of the α-tocopherol content after 6 months of storage (40°C/75% r.h.) of only 14% (I 1-1) up to a maximum decrease of 17% (I 1-2), all comparative examples not containing zinc exhibited a decrease of the α-tocopherol content of at least 20% (C 2-3 and C 1-2) up to 31% (C 2-2), relative to the respective content of α-tocopherol at the start of the storage.

### Example 3:

The correlation was determined between the breaking strength of the pharmaceutical dosage form and the polyalkylene oxide (irrespective of containing zinc or not).

Mixtures of pharmacologically active ingredient, polyalkylene oxide (not containing zinc) and excipients (Hypromellose and PEG 6000) were extruded by means of a twin-screw-extruder (type ZSE 18 PH 40 D, diameter of extrusion die 9 mm, temperature of extrusion die 120-130°C).

The composition of the individual mixtures, the respective extrusion conditions and the breaking strengths of the thus obtained dosage forms are summarized in the table here below:

| Ex. | composition | | revolution velocity [rpm] | throughput [g/min] | breaking strength [N] |
|---|---|---|---|---|---|
| | ingredient | [wt.-%] | | | |
| 1 [5%] | Polyethylene oxide (M_{w} 5 x 10⁶) | 5.00 | 120 | 16.66 | 235 |
| | Tramadol HCl | 50.00 | | | |
| | Hypromellose (M_{w} 1 x 10⁵) | 30.00 | | | |
| | PEG 6000 | 15.00 | | | |
| 2 [15%] | Polyethylene oxide (M_{w} 5 x 10⁶) | 15.00 | 100 | 20 | 785 |
| | Tramadol HCl | 45.00 | | | |
| | Hypromellose (M_{w} 1 x 10⁵) | 30.00 | | | |
| | PEG 6000 | 10.00 | | | |
| 3 [35%] | Polyethylene oxide (M_{w} 5 x 10⁶) | 35.00 | 100 | 33.33 | > 1500 |
| | Tramadol HCl | 45.00 | | | |
| | Hypromellose (M_{w} 1 x 10⁵) | 10.00 | | | |
| | PEG 6000 | 10.00 | | | |
| 4 [55%] | Polyethylene oxide (M_{w} 5 x 10⁶) | 55.00 | 100 | 33.33 | > 1500 |
| | Tramadol HCl | 25.00 | | | |
| | Hypromellose (M_{w} 1 x 10⁵) | 10.00 | | | |
| | PEG 6000 | 10.00 | | | |
| 5 [80%] | Polyethylene oxide (M_{w} 5 x 10⁶) | 80.00 | 100 | 33.33 | > 1500 |
| | Tramadol HCl | 5.00 | | | |
| | Hypromellose (M_{w} 1 x 10⁵) | 5.00 | | | |
| | PEG 6000 | 10.00 | | | |
| 6 | Polyethylene oxide (M_{W} 7 x 10⁶) | 55.00 | 100 | 33.33 | > 1500 |
| | Tramadol HCl | 24.90 | | | |
| [55%] | Hypromellose (M_{W} 1 x 10⁵) | 10.00 | | | |
| | PEG 6000 | 10.00 | | | |

The above data demonstrate that under the given experimental conditions high breaking strengths could only be achieved at contents of polyethylene oxide (5 x 10⁶ and 7 x 10⁶) of 15 wt.-% and above.

## Claims

1. A thermoformed, tamper-resistant pharmaceutical dosage form comprising
a) a pharmacologically active ingredient;
b) a polyalkylene oxide having a weight average molecular weight of more than 200,000 g/mol; and
c) a zinc component, wherein the content of said zinc component is at least 1 parts per million by weight, relative to the total weight of the pharmaceutical dosage form;
which has a breaking strength of at least 300 N, measured in accordance with the Eur. Ph. 6.0, 2.09.08.

2. The pharmaceutical dosage form according to claim 1, which contains a polyalkylene oxide composition including the polyalkylene oxide and the zinc component, and/or wherein the content of said zinc component is in the range of from 0.01 to 1 mol-% based on the zinc atom content per mol of the alkylene oxide units contained in the polyalkylene oxide.

3. The pharmaceutical dosage form according to claim 2, wherein the polyalkylene oxide is obtainable by polymerizing alkylene oxide in presence of the zinc component.

4. The pharmaceutical dosage form according to claim 2 or 3, wherein an aqueous dispersion of the pure polyalkylene oxide composition in pure water at 25°C and at a concentration of 1 wt.-% has a pH value of at most 7.7.

5. The pharmaceutical dosage form according to any of claims 2 to 4, wherein the content of said zinc component is at least 10 parts per million by weight, relative to the total weight of the polyalkylene oxide composition.

6. The pharmaceutical dosage form according to any of the preceding claims, wherein the content of the zinc component is at most 10,000 parts per million by weight, relative to the total weight of the pharmaceutical dosage form.

7. The pharmaceutical dosage form according to any of the preceding claims, wherein the pharmacologically active ingredient is an opioid.

8. The pharmaceutical dosage form according to any of the preceding claims, wherein the pharmacologically active ingredient is embedded in a controlled-release matrix comprising the polyalkylene oxide and the zinc component.

9. The pharmaceutical dosage form according to any of the preceding claims, which is monolithic or multiparticulate.

10. The pharmaceutical dosage form according to any of the preceding claims, which is melt-extruded.

11. The pharmaceutical dosage form according to any of the preceding claims, which is adapted for administration once daily, twice daily or thrice daily.

12. A process for the manufacture of a pharmaceutical dosage form according to any of the preceding claims comprising the steps of
(a) mixing a pharmacologically active ingredient, a polyalkylene oxide composition as defined in any of claims 2 to 5, and optionally present excipients; and
(b) press-forming the mixture obtained in step (a) with preceding, simultaneous, or subsequent exposure to heat.

13. The process according to claim 12, wherein step (b) is performed by means of an extruder.

## Patentansprüche

1. Eine thermogeformte, missbrauchssichere pharmazeutische Darreichungsform umfassend
a) einen pharmazeutisch aktiven Inhaltsstoff;
b) ein Polyalkylenoxid, das ein gewichtsmittleres Molekulargewicht von mehr als 200,000 g/mol aufweist; und
c) einen Zinkbestandteil, wobei der Anteil des Zinkbestandteils wenigstens 1 ppm an Gewicht, bezogen auf das Gesamtgewicht der pharmazeutischen Darreichungsform, ist;
die eine Bruchfestigkeit von wenigstens 300 N aufweist, gemessen in Übereinstimmung mit Pharm. Eur. 6.0, 2.09.08.

2. Die pharmazeutische Darreichungsform gemäß Anspruch 1, die eine Polyalkylenoxidzusammensetzung enthält umfassend das Polyalkylenoxid und den Zinkbestandteil and/oder wobei der Anteil des Zinkbestandteils im Bereich von ab 0.01 bis 1 Mol-% liegt, bezogen auf den Anteil Zinkatome pro Mol der Alkylenoxideinheiten, die im Polyalkylenoxid enthalten sind.

3. Die pharmazeutische Darreichungsform gemäß Anspruch 2, wobei das Polyalkylenoxid durch Polymerisation von Alkylenoxid in der Gegenwart des Zinkbestandteils erhältlich ist.

4. Die pharmazeutische Darreichungsform gemäß Anspruch 2 oder 3, wobei eine wässrige Dispersion der reinen Polyalkylenoxidzusammensetzung in reinem Wasser bei 25°C und einer Konzentration von 1 Gew.-% einen pH-Wert von höchstens 7,7 aufweist.

5. Die pharmazeutische Darreichungsform gemäß einem der Ansprüche 2 bis 4, wobei der Anteil des Zinkbestandteils wenigstens 10 ppm an Gewicht ist, bezogen auf das Gesamtgewicht der Polyalkylenoxidzusammensetzung.

6. Die pharmazeutische Darreichungsform gemäß einem der vorhergehenden Ansprüche, wobei der Anteil des Zinkbestandteils höchstens 10,000 ppm an Gewicht ist, bezogen auf das Gesamtgewicht der Polyalkylenoxidzusammensetzung.

7. Die pharmazeutische Darreichungsform gemäß einem der vorhergehenden Ansprüche, wobei der pharmazeutisch aktive Inhaltsstoff ein Opioid ist.

8. Die pharmazeutische Darreichungsform gemäß einem der vorhergehenden Ansprüche, wobei der pharmazeutisch aktive Inhaltsstoff in einer kontrolliert freigebenden Matrix eingeschlossen ist, die das Polyalkylenoxid und den Zinkbestandteil umfasst.

9. Die pharmazeutische Darreichungsform gemäß einem der vorhergehenden Ansprüche, die monolithisch oder multipartikulär ist.

10. Die pharmazeutische Darreichungsform gemäß einem der vorhergehenden Ansprüche, die schmelzextrudiert ist.

11. Die pharmazeutische Darreichungsform gemäß einem der vorhergehenden Ansprüche, die an die einmal, zweimal oder dreimal tägliche Verabreichung angepasst ist.

12. Ein Verfahren zur Herstellung einer pharmazeutischen Darreichungsform gemäß einem der vorhergehenden Ansprüche umfassend die Schritte
(a) Vermischen eines pharmazeutisch aktiven Inhaltsstoffs, einer Polyalkylenoxidzusammensetzung, wie in den Ansprüchen 2 bis 5 definiert, and gegebenenfalls vorhandener Hilfsstoffe; und
(b) Pressformen der Mischung, die in Schritt (a) erhalten wurde, unter anschließender, gleichzeitiger oder nachfolgender Hitzebehandlung.

13. Das Verfahren gemäß Anspruch 12, wobei Schritt (b) mithilfe eines Extruders durchgeführt wird

## Revendications

1. Forme galénique pharmaceutique thermoformée résistante aux violations comprenant
a) un principe actif pharmacologique ;
b) un oxyde de polyalkylène présentant une masse moléculaire moyenne en poids de plus de 200 000 g/mol ; et
c) un composant à base de zinc, où la teneur en ledit composant à base de zinc est d'au moins 1 partie par million en masse, par rapport à la masse totale de la forme galénique pharmaceutique ;
qui présente une résistance à la rupture d'au moins 300 N, mesurée conformément à Eur. Ph. 6.0, 2.09.08.

2. Forme galénique pharmaceutique selon la revendication 1, qui contient une composition d'oxyde de polyalkylène incluant un polyoxyde d'alkylène et le composant à base de zinc, et/ou où la teneur en ledit composant à base de zinc est comprise dans l'intervalle allant de 0,01 à 1 % molaire par rapport à la teneur en atomes de zinc par mole de motifs oxyde d'alkylène contenus dans le polyoxyde d'alkylène.

3. Forme galénique pharmaceutique selon la revendication 2, où le polyoxyde d'alkylène peut être obtenu par polymérisation de l'oxyde d'alkylène en présence du composant à base de zinc.

4. Forme galénique pharmaceutique selon la revendication 2 ou 3, où une dispersion aqueuse de la composition de polyoxyde d'alkylène pure dans l'eau pure à 25 °C et à une concentration de 1 % en masse présente une valeur de pH maximale de 7,7.

5. Forme galénique pharmaceutique selon l'une quelconque des revendications 2 à 4, où la teneur en ledit composant à base de zinc est d'au moins 10 parts par million en masse, par rapport à la masse totale de la composition de polyoxyde d'alkylène.

6. Forme galénique pharmaceutique selon l'une quelconque des revendications précédentes, où la teneur en ledit composant à base de zinc est d'au moins 10 000 parts par million en masse, par rapport à la masse totale de la forme galénique pharmaceutique.

7. Forme galénique pharmaceutique selon l'une quelconque des revendications précédentes, où le principe actif pharmacologique est un opioïde.

8. Forme galénique pharmaceutique selon l'une quelconque des revendications précédentes, où le principe actif pharmacologique est intégré dans une matrice à libération contrôlée comprenant le polyoxyde d'alkylène et le composant à base de zinc.

9. Forme galénique pharmaceutique selon l'une quelconque des revendications précédentes, qui est monolithique ou multiparticulaire.

10. Forme galénique pharmaceutique selon l'une quelconque des revendications précédentes, qui est extrudée à l'état fondu.

11. Forme galénique pharmaceutique selon l'une quelconque des revendications précédentes, qui est adaptée à une administration une fois par jour, deux fois par jour ou trois fois par jour.

12. Procédé de fabrication d'une forme galénique pharmaceutique selon l'une quelconque des revendications précédentes comprenant les étapes suivantes :
(a) mélangeage d'un principe actif pharmacologique, d'une composition de polyoxyde d'alkylène selon l'une quelconque des revendications 2 à 5 et d'excipients éventuellement présents ; et
(b) façonnage par pressage du mélange obtenu dans l'étape (a) avec exposition antérieure, simultanée ou ultérieure à la chaleur.

13. Procédé selon la revendication 12, où l'étape (b) est mise en oeuvre par le biais d'une extrudeuse.
